# EUROPEAN PATENT APPLICATION

(11) **EP 2 065 395 A1**
(43) Date of publication of application: **03.06.2009**
(21) Application number: 09151973.6
(22) Date of filing: 05.04.2004
(51) Int. Cl.: C07K 14/47

(54) **Gene expression associated with osteoblast differentiation**

(30) Priority: 14.04.2003 US 462834 P
(62) Divisional of application: 07119293.4
(71) Applicant: NOVARTIS AG, 4002 Basel (CH)
(72) Inventor: Susa Spring, Mira, 4055, Basel (CH); Zamurovic, Natasa, 4057, Basel (CH)
(74) Representative: Spinner, David Richard

(57) **Abstract**

The present invention identifies genes whose expression pattern is altered when pre-osteoblastic cells undergo differentiation into mature osteoblasts. The genes identified may be used as markers for the differentiation process. The present invention also provides methods to screen agents that are capable of modulating the differentiation process. The present invention also provides methods of identifying therapeutic agents that stimulate bone formation by analyzing the expression of one or more of the genes identified.

SEQUENCE LISTING

<110>  Novartis AG

<120>  Gene expression associated with osteoblast differentiation

<130>  PA/A-32899A

<160>  52

<170>  PatentIn version 3.3

<210>  1
<211>  19
<212>  DNA
<213>  artificial

<220>
<223>  Primer

<400>  1
cccaaaggct tcttcttgc                                                    19


<210>  2
<211>  19
<212>  DNA
<213>  artificial

<220>
<223>  primer

<400>  2
gcctggtagt tgttgtgag                                                    19


<210>  3
<211>  19
<212>  DNA
<213>  artificial

<220>
<223>  primer

<400>  3
cgcctcggtc aagtcggaa                                                    19


<210>  4
<211>  19
<212>  DNA
<213>  artificial

<220>
<223>  primer

<400>  4
gcccgctctg ctagtgaca                                                    19


<210>  5
<211>  20
<212>  DNA

<213>  artificial

<220>
<223>  primer

<400>  5
accccgagtc taaagagaac                                                    20


<210>  6
<211>  20
<212>  DNA
<213>  artificial

<220>
<223>  primer

<400>  6
gcctttgtgg ttgaagtcat                                                    20


<210>  7
<211>  21
<212>  DNA
<213>  artificial

<220>
<223>  primer

<400>  7
gggcaataag gtagtgaaca g                                                  21


<210>  8
<211>  21
<212>  DNA
<213>  artificial

<220>
<223>  primer

<400>  8
gcagcacagg tcctaaatag t                                                  21


<210>  9
<211>  18
<212>  DNA
<213>  artificial

<220>
<223>  primer

<400>  9
atgcttcatt cgcctcac                                                      18


<210>  10
<211>  18
<212>  DNA
<213>  artificial

<220>

<223>  primer

<400>  10
ctcacgtcgc tcatcttg                                                    18


<210>  11
<211>  21
<212>  DNA
<213>  artificial

<220>
<223>  primer

<400>  11
cacaagcaga cactttcact c                                                21


<210>  12
<211>  21
<212>  DNA
<213>  artificial

<220>
<223>  primer

<400>  12
gaatgctcaa gtctgtgtgt t                                                21


<210>  13
<211>  20
<212>  DNA
<213>  artificial

<220>
<223>  primer

<400>  13
ccctgccaga accatcattg                                                  20


<210>  14
<211>  20
<212>  DNA
<213>  artificial

<220>
<223>  primer

<400>  14
ttgcatggtc cgatgtagtc                                                  20


<210>  15
<211>  20
<212>  DNA
<213>  artificial

<220>
<223>  primer

<400>  15

ccctgcctgc ttcgtgtaaa                                                20

<210>  16
<211>  22
<212>  DNA
<213>  artificial

<220>
<223>  primer

<400>  16
ccaaagtcca tgtgaaatta tc                                              22

<210>  17
<211>  20
<212>  DNA
<213>  artificial

<220>
<223>  primer

<400>  17
cctggatacc gcagctagga                                                20

<210>  18
<211>  21
<212>  DNA
<213>  artificial

<220>
<223>  primer

<400>  18
gcggcgcaat acgaatgccc c                                               21

<210>  19
<211>  20
<212>  DNA
<213>  artificial

<220>
<223>  primer

<400>  19
ctgcaccacc aactgcttag                                                20

<210>  20
<211>  20
<212>  DNA
<213>  artificial

<220>
<223>  primer

<400>  20
agatccacga cggacacatt                                                20

<210>  21
<211>  20
<212>  DNA
<213>  artificial

<220>
<223>  primer

<400>  21
tgctggtgga tggtttcaca                                                    20


<210>  22
<211>  21
<212>  DNA
<213>  artificial

<220>
<223>  primer

<400>  22
tgtcgcctgg tgttttcaat a                                                  21


<210>  23
<211>  19
<212>  DNA
<213>  artificial

<220>
<223>  primer

<400>  23
gcaaccccta ccacttcag                                                     19


<210>  24
<211>  20
<212>  DNA
<213>  artificial

<220>
<223>  primer

<400>  24
gcctcggttt cagtgtaaga                                                    20


<210>  25
<211>  20
<212>  DNA
<213>  artificial

<220>
<223>  primer

<400>  25
cagcctttct atcacgacga                                                    20


<210>  26
<211>  20
<212>  DNA

<213>  artificial

<220>
<223>  primer

<400>  26
ggtgggtttc aggagtttgt                                                    20


<210>  27
<211>  20
<212>  DNA
<213>  artificial

<220>
<223>  primer

<400>  27
ccgatgagtc tgctctacaa                                                    20


<210>  28
<211>  19
<212>  DNA
<213>  artificial

<220>
<223>  primer

<400>  28
ccgtgttcag ggtggtcag                                                     19


<210>  29
<211>  20
<212>  DNA
<213>  artificial

<220>
<223>  primer

<400>  29
aaaccacgca ccatctactc                                                    20


<210>  30
<211>  20
<212>  DNA
<213>  artificial

<220>
<223>  primer

<400>  30
tcgccgcttt tccacatctt                                                    20


<210>  31
<211>  19
<212>  DNA
<213>  artificial

<220>

<223>  primer

<400>  31
accgcccagc agcagaagt                                                    19


<210>  32
<211>  19
<212>  DNA
<213>  artificial

<220>
<223>  primer

<400>  32
aggtcgggga tagccagtg                                                    19


<210>  33
<211>  18
<212>  DNA
<213>  artificial

<220>
<223>  primer

<400>  33
ccgacgctgg aactgctc                                                     18


<210>  34
<211>  20
<212>  DNA
<213>  artificial

<220>
<223>  primer

<400>  34
atggaacagg cttgagtgac                                                   20


<210>  35
<211>  20
<212>  DNA
<213>  artificial

<220>
<223>  primer

<400>  35
actctgcctt caatctgctc                                                   20


<210>  36
<211>  20
<212>  DNA
<213>  artificial

<220>
<223>  primer

<400>  36

gggtgtggac tgctgaaatg                                                    20

<210>  37
<211>  20
<212>  DNA
<213>  artificial

<220>
<223>  primer

<400>  37
gccagtgtgt cctcatcaag                                                    20

<210>  38
<211>  20
<212>  DNA
<213>  artificial

<220>
<223>  primer

<400>  38
acgctggcag acaaagtaga                                                    20

<210>  39
<211>  21
<212>  DNA
<213>  artificial

<220>
<223>  primer

<400>  39
ccaaagacat ctcacacagt a                                                  21

<210>  40
<211>  20
<212>  DNA
<213>  artificial

<220>
<223>  primer

<400>  40
tgccgtacaa ctccagtgac                                                    20

<210>  41
<211>  19
<212>  DNA
<213>  artificial

<220>
<223>  primer

<400>  41
caccgctgaa tggctgtct                                                     19

<210>  42
<211>  20
<212>  DNA
<213>  artificial

<220>
<223>  primer

<400>  42
cattgggctg aaaggtgtga                                                    20


<210>  43
<211>  20
<212>  DNA
<213>  artificial

<220>
<223>  primer

<400>  43
ttcagcagca agggtcactc                                                    20


<210>  44
<211>  21
<212>  DNA
<213>  artificial

<220>
<223>  primer

<400>  44
gacaggcaaa cttcttctca c                                                  21


<210>  45
<211>  20
<212>  DNA
<213>  artificial

<220>
<223>  primer

<400>  45
gccgacgaga ccgaatcaat                                                    20


<210>  46
<211>  20
<212>  DNA
<213>  artificial

<220>
<223>  primer

<400>  46
gctgggatgc gtagttgttg                                                    20


<210>  47
<211>  20
<212>  DNA

<213>  artificial

<220>
<223>  primer

<400>  47
gctgccatcg tgaaagagaa                                                    20


<210>  48
<211>  20
<212>  DNA
<213>  artificial

<220>
<223>  primer

<400>  48
tccacggctt tgttgtactc                                                    20


<210>  49
<211>  19
<212>  DNA
<213>  artificial

<220>
<223>  primer

<400>  49
tctggctgct gctggctca                                                     19


<210>  50
<211>  20
<212>  DNA
<213>  artificial

<220>
<223>  primer

<400>  50
tttccccagt cacaccatag                                                    20


<210>  51
<211>  20
<212>  DNA
<213>  artificial

<220>
<223>  primer

<400>  51
cgggacagtt cttcgcatct                                                    20


<210>  52
<211>  19
<212>  DNA
<213>  Artificial

<220>

<223>  Primer

<400>  52
ttcggtctgg cttggtcac                                                    19

## Description

The present invention identifies genes, e.g. Hey1, whose expression pattern is altered when precursor pre-osteoblastic cells undergo differentiation into osteoblasts. The genes identified may be used as markers for the differentiation process. The present invention also provides methods to screen agents that are capable of modulating the differentiation process. The present invention also provides methods of identifying therapeutic agents that stimulate bone formation by analyzing the expression of one or more of the genes identified.

### Background of the invention

Bone is a dynamic tissue in which old tissue is broken down and new tissue is synthesized. Control of the rate of breakdown and synthesis of new bone tissue is critical to the integrity of the skeletal structure. When the rates become unbalanced, serious conditions may result. The process of synthesizing new bone tissue is mediated by osteoblasts. During the process of synthesizing new bone tissue, osteoblasts differentiate from precursor pre-osteoblastic cells to mature bone-forming cells. Osteoblast differentiation process was previously examined mostly on cellular or single gene levels. Many external regulating factors are known, such as TGFb family members, but critical molecular steps in osteoblast differentiation and bone formation are largely unknown. One key player was identified recently as the transcription factor Cbfa1 (reviewed by Karsenty, 2000, Semin Cell Dev Biol; 11: 343-346). Another transcription factor, Osterix (Osx), which cooperates with and is genetically downstream of Cbfa1, has also been identified (Nakashima et al., 2002, Cell; 108:17-29). The expression levels of various enzymes and structural proteins, for example alkaline phosphatase and type-1 collagen, are up-regulated, while other genes are down-regulated. In order to treat a condition characterized by an imbalance in the rates of breakdown and synthesis of bone tissue, it may be desirable to increase or decrease the rate of break down and/or synthesis. Thus, in a number of clinical applications, it may desirable to enhance the rate of bone formation by promoting the differentiation of precursor pre-osteoblastic cells into osteoblasts. One application that is particularly important is the treatment of osteoporosis, characterized by a decrease in bone mass making the bones more fragile and subject to fracture. Other potential uses for reagents capable of affecting the synthesis of bone tissue include the healing of broken bones, recovery after surgical procedures involving bones and the like. While the changes in the expression levels of a number of individual genes have been identified, the investigation of the global changes in gene expression, which occur in precursor stem cells as they differentiate into osteoblasts has been reported elsewhere (Qi et al., PNAS USA, 2003, 100(6), 3305-10; de Jong et al., J Bone Miner Res. 2002, 17(12): 2119-29; Vaes et al., J Bone Miner Res. 2002, 17(12):2106-18) but do not overlap (or only partially) with the finding presented in this study. However accurate information would be useful, for example, in assessing the effects of a course of treatment designed to change the rate of formation of bone tissue. Accordingly, there exists a need for the investigation of the changes in global gene expression levels, as well as the need for the identification of new molecular markers associated with the differentiation of precursor cells into osteoblasts. Furthermore, identification of additional genes involved in differentiation may allow development of reagents designed to alter their expression levels and thereby allow control of the differentiation process. In addition, identification of the genes involved in the process allows their use as diagnostic or prognostic markers which are uniquely associated with differentiation.

Bone forming osteoblasts develop from their mesenchymal precursors in vivo and in vitro. Different cellular phenotypes during osteogenesis were tentatively defined as osteoprogenitors, pre-osteoblasts, mature osteoblasts and mineralizing osteoblasts, each of them characterized by an overlapping yet distinct set of marker genes (Aubin et al., 1995, Bone; 17: 77S-83S). Cytochemically, classical markers of osteoblast differentiation are stainings for alkaline phosphatase and for mineralized bone nodules in culture. In order to examine osteoblast differentiation in vitro, a crucial step is the use of appropriate primary cells or a cell line and defined culture conditions, which allow an ordered step-wise differentiation process. This is difficult to achieve with osteosarcoma cell lines, many of which are available from human and rat origin (SaOS, U2OS, ROS, and UMR). This is because due to their tumor origin, these cell lines show de-regulated proliferation, and often express differentiation markers together with proliferation markers. In addition, these cell lines rarely exhibit formation of mineralized bone nodules in vitro, a hallmark of completed osteogenesis. In order to avoid these inherent problems with differentiation of osteosarcoma cell lines, a need for new models is imminent.

### Summary of the invention

The present invention relates to the elucidation of the global changes in gene expression during osteoblastic differentiation of MC3T3-E1 cell line, in particular MC3T3-1b clone. In one aspect, the present invention relates to detecting a change in an expression level of one or more genes or gene families associated with the differentiation of MC3T3-E1 cells, in particular MC3T3-1b cells, into osteoblasts. In a related aspect, the activity of a protein encoded by a gene or member of a gene family may be assayed. Such assays may be conducted by themselves or in conjunction with determining an expression level. In some aspects, it may be desirable to determine an expression level of one or more genes or members of a gene family in Table 1 or 2, while at the same time determining an activity level of one or more proteins encoded by a gene or member of a gene family of Table 1 or 2. The genes or member of gene families for which expression levels are determined may be the same or different as the genes encoding the proteins assayed. Thus, in some embodiments, it may be desirable to determine the expression level of a gene and the activity level of the protein encoded by the gene. In other embodiments, it may be desirable to determine the expression level of one gene while determining the activity level of a protein encoded by another gene. Those skilled in the art will appreciate that the expression and/or activity-level of any number of genes and proteins may be determined according to the present invention. In a related aspect, the present invention includes methods of validating a gene or gene family found above, i.e. confirming the gene's or protein's role in e.g. osteoporosis.

In a related aspect, the present invention includes methods of screening for an agent that modulates the differentiation of a MC3T3-E1 cell line, in particular MC3T3-1 b clone, into an osteoblast, comprising: preparing a first gene or gene family expression profile and/or assaying for an activity of a protein encoded by a gene or member of a gene family of Table 1 or 2 in a cell population comprising one or more MC3T3-E1 cells, in particular MC3T3-1 b cells; contacting the cell population with an agent; preparing a second gene or gene family expression profile and/or assaying for an activity of a protein encoded by a gene or member of a gene family of Tables 1 or 2 of the cell population after being contacted with the agent; and comparing the first and second expression profiles and/or activities.

In one aspect, the present invention provides a method of diagnosing a condition characterized by abnormal deposition of bone tissue, comprising detecting the level of expression in a tissue sample of one or more genes or gene families from Table 1 or 2 and/or assaying for an activity of a protein encoded by a gene or member of a gene family of Table 1 or 2, wherein differential expression and/or activity is indicative of inadequate bone tissue deposition. The invention includes methods of diagnosing a condition characterized by an abnormal rate of formation of osteoblasts in a patient comprising detecting the level of expression in a tissue sample of one or more genes or gene families from Table 1 or 2 and/or assaying for an activity of a protein encoded by a gene or member of a gene family of Table 1 or 2; wherein differential expression and/or activity is indicative of an abnormal rate of formation of osteoblasts.

The invention further includes a method of diagnosing osteoporosis in a patient comprising detecting the level of expression in a tissue sample of one or more genes or gene families from Table 1 or 2 and/or assaying for an activity of a protein encoded by a gene or member of a gene family of Table 1 or 2; wherein differential expression and/or activity is indicative of osteoporosis.

In another aspect, the present invention also includes methods of monitoring the treatment of a patient with a condition characterized by abnormal bone tissue deposition, comprising administering a pharmaceutical composition to the patient, preparing a gene or gene family expression profile and/or assaying for an activity of a protein encoded by a gene or member of a gene family of Table 1 or 2 from a cell or tissue sample from the patient and comparing the patient expression profile and/or activity to an expression profile and/or activity from a MC3T3-E1, in particular MC3T3-1 b, cell population or an osteoblast cell population.

In a related aspect, the present invention provides a method of monitoring the treatment of a patient with osteoporosis, comprising administering a pharmaceutical composition to the patient, preparing a gene or gene family expression profile and/or assaying for an activity of a protein encoded by a gene or member of a gene family of Table 1 or 2 from a cell or tissue sample from the patient and comparing the patient expression profile and/or activity to an expression profile and/or activity from a MC3T3-E1, in particular MC3T3-1 b, cell population or an osteoblast cell population.

In one aspect, the present invention is a method of monitoring the progression of bone tissue deposition in a patient, comprising detecting the level of expression in a tissue sample of one or more genes or gene families from Table 1 or 2 and/or assaying for an activity of a protein encoded by a gene or member of a gene family of Table 1 or 2; wherein differential expression and/or activity is indicative of bone tissue deposition.

The invention includes a method of monitoring the treatment of a patient with a condition characterized by abnormal rate of formation of osteoblasts, comprising administering a pharmaceutical composition to the patient, preparing a gene or gene family expression profile and/or assaying for an activity of a protein encoded by a gene or member of a gene family of Table 1 or 2 from a cell or tissue sample from the patient and comparing the patient expression profile and/or activity to an expression profile and/or activity from a MC3T3-E1, in particular MC3T3-1 b, cell population or an osteoblast cell population.

In a related aspect, the present invention provides a method of treating a patient with a condition characterized by an abnormal rate of formation of osteoblasts, comprising administering to the patient a pharmaceutical composition, wherein the composition alters the expression of at least one gene or gene family in Table 1 or 2 and/or alters an activity of a protein encoded by a gene or member of a gene family of Table 1 or 2, preparing an expression profile and/or assaying for an activity from a MC3T3-E1, in particular MC3T3-1 b, cell and comparing the patient expression profile and/or activity to an expression profile and/or activity from an untreated cell population comprising MC3T3-E1, in particular MC3T3-1 b, cells.

In another aspect, the present invention also includes methods of treating a patient with a condition characterized by abnormal bone tissue deposition, comprising administering a pharmaceutical composition to the patient; preparing a gene or gene family expression profile and/or assaying for an activity of a protein encoded by a gene or member of a gene family of Table 1 or 2 from a cell or tissue sample from the patient comprising MC3T3-E1 cells, in particular MC3T3-1 b cells; and comparing the patient expression profile and/or activity to an expression profile and/or activity from an untreated cell population comprising MC3T3-E1 cells, in particular MC3T3-1 b cells.

In one aspect, the present invention provides a method of treating a patient with osteoporosis, comprising administering to the patient a pharmaceutical composition, wherein the composition alters the expression of at least one gene or gene family in Table 1 or 2 and/or alters an activity of a protein encoded by a gene or member of a gene family of Table 1 or 2, preparing an expression profile and/or assaying for an activity of a protein encoded by a gene or member of a gene family of Table 1 or 2 from a MC3T3-E1 cell, in particular MC3T3-1 b cell, and comparing the patient expression profile and/or activity to an expression profile and/or activity from an untreated cell population comprising MC3T3-E1, in particular MC3T3-1 b, cells.

Also included in the inventions are methods of screening for an agent capable of ameliorating the effects of osteoporosis, comprising exposing a cell to the agent; and detecting the expression level of one or more genes or gene families from Table 1 or 2 and/or assaying for an activity of a protein encoded by a gene or member of a gene family of Table 1 or 2.

In a further aspect, the present invention is a method of screening for an agent capable of modulating the deposition of bone tissue, comprising exposing a cell to the agent and detecting the expression level of one or more genes or gene families from Table 1 or 2 and/or assaying for an activity of a protein encoded by a gene or member of a gene family of Table 1 or 2.

All of these methods may include the step of detecting the expression levels of at least 1, 2, 3, 4, 5, 6, 7, 8, 9, 10 or more genes or members of the gene families in Table 1 or 2. Preferably, expression of all of the genes or members of the gene families or nearly all of the genes or members of the gene families in Table 1 or 2 may be detected. In a related aspect, the methods of the present invention may comprise the step of assaying for an activity of at least 1, 2, 3, 4, 5, 6, 7, 8, 9, 10 or more proteins encoded by a gene or member of a gene family of Table 1 or 2. In some preferred embodiments, the methods of the present invention may comprise both determining an expression level of one or more genes of members of a gene family of Table 1 or 2 and assaying an activity of one or more proteins encoded by a gene or member of a gene family of Table 1 or 2. In some embodiments, the expression level of a gene and the activity level of the protein encoded by the same gene may be determined. In other embodiments, the expression level of at least one gene may be determined while the activity level of at least one protein encoded by a different gene may be determined.

In one aspect, the present invention provides a method for identifying an agent that modulates the differentiation of MC3T3-E1, in particular MC3T3-1b, cells into osteoblasts comprising contacting a cell population with the agent and assaying for at least one activity of at least one gene or the activity of at least one member of a gene family identified in Table 1 or 2. In a related aspect, the present invention provides a method of monitoring the treatment of a patient with a condition characterized by abnormal bone deposition comprising administering a pharmaceutical composition to the patient and assaying for at least one activity of at least one gene or one member of a gene family identified in Table 1 or 2. The present invention also includes a method of diagnosing a condition characterized by the abnormal rate of formation of osteoblast comprising detecting the level of activity of at least one gene or one member of a gene family identified in Table 1 or 2. In some preferred aspects, the present invention encompasses a composition comprising at least two oligonucleotides, wherein each of the oligonucleotides comprises a sequence that specifically hybridizes to one or more genes or members of a gene family in Table 1 or 2. In some aspects, the composition may comprise at least 1, 2, 3, 4, 5, 6, 7, 8, 9, 10 oligonucleotides, wherein each of the oligonucleotides comprises a sequence that specifically hybridizes to one or more genes or members of a gene family in Table 1 or 2. In some embodiments, one or more of the oligonucleotides may be attached to a solid support. The solid support may be any known to those skilled in the art including, but not limited to, a membrane, a glass support, a filter, a tissue culture dish, a polymeric material and a silicon support.

In a preferred aspect, the present invention provides a solid support to which is attached at least two oligonucleotides, wherein each of the oligonucleotides comprises a sequence that specifically hybridizes to at least one gene or to at least one member of a gene family in Table 1 or 2. In some embodiments, at least one oligonucleotide is attached covalently to the solid support. In some embodiments, at least one oligonucleotide is attached non-covalently to the solid support. Oligonucleotides may be attached to the solid supports of the invention at any density known to those skilled in the art, for example, at about at least 10 different oligonucleotides in discrete locations per square centimeter, at about at least 100 different oligonucleotides in discrete locations per square centimeter, at about at least 1000 different oligonucleotides in discrete locations per square centimeter and/or at about at least 10,000 different oligonucleotides in discrete locations per square centimeter. The selection of an appropriate density for a given application is a routine procedure for those skilled in the art.

The invention also includes computer systems comprising a database containing information identifying the expression level of one or more members of one or more of the gene families in Table 1 or 2 and/or the activity level of one or more proteins encoded by a gene or by a member of a gene family of Table 1 or 2 in a resting MC3T3-E1, in particular MC3T3-1 b, cell and/or a MC3T3-E1, in particular MC3T3-1 b, cell differentiating into an osteoblast and/or an osteoblast; and a user interface to view the information. The database may further comprise sequence information for one or more of the genes of one or one or more members of one or more of the gene families of Table 1 or 2. The database may comprise information identifying the expression level for one or more genes or one or more members of one or more of the gene families in the set of gene families expressed in a MC3T3-E1, in particular MC3T3-1 b, cell that is not differentiating. The database may comprise information identifying the expression level for one or more genes or one or more members of one or more of the gene families in the set of genes or gene families expressed in a MC3T3-E1, in particular MC3T3-1 b, cell that is differentiating into a cell type other than an osteoblast. The database may comprise information identifying the expression level for one or more genes or one or more members of one or more of the gene families in the set of genes or gene families expressed in a precursor stem cell that is differentiating into an osteoblast. The database may further contain or be linked to descriptive information from an external database, which information correlates said genes and/or gene families to records in the external database.

Moreover, the invention includes methods of using the disclosed computer systems to present information identifying the expression level in a tissue or cell of a set of genes and/or gene families comprising at least one of the genes or gene families in Table 1 or 2, comprising comparing the expression level of at least one gene or gene family in Table 1 or 2 in the tissue or cell to the level of expression of the gene in the database.

The invention also includes methods of using the disclosed computer systems to present information identifying the activity level in a tissue or cell of one or more proteins encoded by one or more genes and/or members of a gene family comprising at least one of the genes or gene families in Table 1 or 2, comprising comparing the activity level of at least one protein encoded by one gene or member of a gene family in Table 1 or 2 in the tissue or cell to the level of activity of the protein in the database.

### Detailed description

Many biological functions are accomplished by altering the expression of various genes through transcriptional (e.g. through control of initiation, provision of RNA precursors, RNA processing, etc.) and/or translational control. For example, fundamental biological processes such as cell cycle, cell differentiation and cell death, are often characterized by the variations in the expression levels of groups of genes. Changes ill gene expression also are associated with pathogenesis. For example, the lack of sufficient expression of functional tumor suppressor genes and/or the overexpression of oncogene/protooncogenes could lead to tumorgenesis or hyperplastic growth of cells (Marshall, (1991) Cell 64:313-326; Weinberg, (1991) Science 254:1138-1146). Thus, changes in the expression levels of particular genes (e.g., oncogenes or tumor suppressors) serve as signposts for the presence and progression of various diseases. Monitoring changes in gene expression may also provide certain advantages during drug screening development. Often drugs are screened for the ability to interact with a major target without regard to other effects the drugs have on cells. Often such other effects cause toxicity in the whole animal, which prevent the development and use of the potential drug.

The present inventors have examined cell populations comprising MC3T3-E1 cells, in particular MC3T3-1 b cells, that have been induced to differentiate into osteoblasts to identify the global changes in gene expression during this differentiation process. These global changes in gene expression, also referred to as expression profiles, provide useful markers for diagnostic uses as well as markers that can be used to monitor disease states, disease progression, toxicity, drug efficacy and drug metabolism.

The expression profiles have been used to identify individual genes that are differentially expressed under one or more conditions. In addition, the present invention identifies families of genes that are differentially expressed. As used herein, "gene families" includes, but is not limited to, the specific genes identified by accession number herein, as well as related sequences. Related sequences may be, for example, sequences having a high degree of sequence identity with a specifically identified sequence either at the nucleotide level or at the level of amino acids of the encoded polypeptide. A high degree of sequence identity is seen to be at least about 65% sequence identity at the nucleotide level to said genes, preferably about 80 or 85% sequence identity or more preferably about 90 or 95% or more sequence identity to said genes. With regard to amino acid identity of encoded polypeptides, a high degree of identity is seen to be at least about 50% identity, more preferably about 75% identity and most preferably about 85% or more sequence identity. In particular, related sequences include homologous genes from different organisms. For example, if the specifically identified gene is from a non-human mammal, the gene family would encompass homologous genes from other mammals including humans. If the specifically identified gene is a human gene, gene family would encompass the homologous gene from different organisms. Those skilled in the art will appreciate that a homologous gene may be of different length and may comprise regions with differing amounts of sequence identity to a specifically identified sequence.

Assav Formats: The genes and sequences identified as being differentially expressed in the cell population induced to differentiate as well as related sequences may be used in a variety of nucleic acid detection assays to detect or quantitate the expression level of a gene or multiple genes in a given sample. For example, traditional Northern blotting, ribonuclease protection, RT-PCR, QPCR (quantitative RT-PCR), real-time PCR such as TaqMan System and differential display methods may be used for detecting gene expression levels. Those methods are useful for some embodiments of the invention. However, methods and assays of the invention are most efficiently designed with hybridization-based methods for detecting the expression of a large number of genes. Any hybridization assay format may be used, including solution-based and solid support-based assay formats. Solid supports containing oligonucleotide probes for differentially expressed genes of the invention can be filters, polyvinyl chloride dishes, silicon or glass based chips, etc. Such supports and hybridization methods are widely available, for example, those disclosed by WO 95/11755. Any solid surface to which oligonucleotides can be bound, either directly or indirectly, either covalently or non covalently, can be used. A preferred solid support is a high density array or DNA chip. These contain a particular oligonucleotide probe in a predetermined location on the array. Each predetermined location may contain more than one molecule of the probe, but each molecule within the predetermined location has an identical sequence. Such predetermined locations are tended features. There may be, for example, from 2, 10, 100, 1000 to 10000, 100000 or 400000 of such features on a single solid support. The solid support, or the area within which the probes are attached may be any convenient size and may preferably be on the order of a square centimeter. Oligonucleotide probe arrays for expression monitoring can be made and used according to any techniques known in the art (see for example, Lockhart et al., (1996) Nat. Biotech. 14, 1675-1680, McGall et al., (1996) Proc. Nat. Acad. Sci. USA 93, 13555-13460). Such probe arrays may contain at least two or more oligonucleotides that are complementary to or hybridize to two or more of the genes described in Table 1 or 2. For instance, such arrays may also contain oligonucleotides that are complementary or hybridize to at least about 1, 2, 3, 4, 5, 6, 7, 8, 9, 10, 20, 30, 50, 70 or more the genes described herein. The genes which are assayed according to the present invention are typically in the form of mRNA or reverse transcribed mRNA. The genes may be cloned or not. The genes may be amplified or not. The cloning itself does not appear to bias the representation of genes within a population. However, it may be preferable to use polyadenylated RNA as a source, as it can be used with less processing steps.

Table 1 and 2 provides the Accession numbers and name for the sequences of the differentially expressed markers. The sequences of the genes in GenBank are expressly incorporated herein.

Probes based on the sequences of the genes described above may be prepared by any commonly available method. Oligonucleotide probes for interrogating the tissue or cell sample are preferably of sufficient length to specifically hybridize only to appropriate, complementary genes or transcripts. Typically the oligonucleotide probes will be at least 10, 12, 14, 16, 18, 20 or 25 nucleotides in length. In some cases longer 20 probes of at least 30, 40 or 50 nucleotides will be desirable.

As used herein, oligonucleotide sequences that are complementary to one or more of the genes and/or gene families described in Table 1 or 2, refer to oligonucleotides that are capable of hybridizing under stringent conditions to at least part of the nucleotide sequences of said genes. Such hybridizable oligonucleotides will typically exhibit at 25 least about 75% sequence identity at the nucleotide level to said genes, preferably about 80 or 85% sequence identity or more preferably about 90 or 95% or more sequence identity to said genes.

"Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The terms "background" or "background signal intensity" refer to hybridization signals resulting from non-specific binding, or other interactions, between the labeled target nucleic acids and components of the oligonucleotide array (e.g. the oligonucleotide probes, control probes, the array substrate, etc.). Background signals may also be produced by intrinsic fluorescence of the array components themselves. A single background signal can be calculated for the entire array, or a different background signal may be calculated for each target nucleic acid. In a preferred embodiment, background is calculated as the average hybridization signal intensity for the lowest 5 to 10% of the probes in the array, or, where a different background signal is calculated for each target gene, for the lowest 5 to 10% of the probes for each gene. Of course, one of skill in the art will appreciate that where the probes to a particular gene hybridize well and thus appear to be specifically binding to a target sequence, they should not be used in a background signal calculation. Alternatively, background may be calculated as the average hybridization signal intensity produced by hybridization to probes that are not complementary to any sequence found in the sample (e.g., probes directed to nucleic acids of the opposite sense or to genes not found in the sample such as bacterial genes where the sample is mammalian nucleic acids). Background can also be calculated as the average signal intensity produced by regions of the array that lack any probes at all.

The phrase "hybridizing specifically to" refers to the binding, duplexing, or hybridizing of a molecule substantially to or only to a particular nucleotide sequence or sequences under stringent conditions when that sequence is present in a complex mixture (e.g. total cellular) DNA or RNA.

Assays and methods of the invention may utilize available formats to simultaneously screen at least about 100, preferably about 1000, more preferably about 25 10,000 and most preferably about 1,000,000 different nucleic acid hybridizations.

The terms "mismatch control" or "mismatch probe" refer to a probe whose sequence is deliberately selected not to be perfectly complementary to a particular target sequence. For each mismatch (MM) control in a high- density array there typically exists a corresponding perfect match (PM) probe that is perfectly complementary to the same particular target sequence. The mismatch may comprise one or more bases.

While the mismatch(s) may be located anywhere in the mismatch probe, terminal mismatches are less desirable as a terminal mismatch is less likely to prevent hybridization of the target sequence. In a particularly preferred embodiment, the mismatch is located at or near the center of the probe such that the mismatch is most likely to destabilize the duplex with the target sequence under the test hybridization conditions.

The term "perfect match probe" refers to a probe that has a sequence that is perfectly complementary to a particular target sequence. The test probe is typically perfectly complementary to a portion (subsequence) of the target sequence. The perfect match (PM) probe can be a "test probe" or a "normalization control" probe, an expression level control probe and the like. A perfect match control or perfect match probe is, however, distinguished from a "mismatch control" or "mismatch probe" as defined herein.

As used herein a "probe" is defined as a nucleic acid, capable of binding to a target nucleic acid of complementary sequence through one or more types of chemical bonds, usually through complementary base pairing, usually through hydrogen bond formation. As used herein, a probe may include natural (i.e., A, G. U. C or T) or modified bases (7-deazaguanosine, inosine, etc.). In addition, the bases in probes may be joined by a linkage other than a phosphodiester bond, so long as it does not interfere with hybridization. Thus, probes may be peptide nucleic acids in which the constituent bases are joined by peptide bonds rather than phosphodiester linkages.

The term "stringent conditions" refers to conditions under which a probe will hybridize to its target subsequence, but with only insubstantial hybridization to other sequences or to other sequences such that the difference may be identified. Stringent conditions are sequence-dependent and will be different in different circumstances. Longer sequences hybridize specifically at higher temperatures. Generally, stringent conditions are selected to be about 5°C lower than the thermal melting point (Tm) for the specific sequence at a defined ionic strength and pH. Typically, stringent conditions will be those in which the salt concentration is at least about 0.01 to 1.0 M sodium ion concentration (or other salts) at pH 7.0 to 8.3 and 30 the temperature is at least about 30°C for short probes (e.g. 10 to 50 nucleotides). Stringent conditions may also be achieved with the addition of destabilizing agents such as formamide.

The 'percentage of sequence identity" or "sequence identity" is determined by comparing two optimally aligned sequences or subsequences over a comparison window or span, wherein the portion of the polynucleotide sequence in the comparison window 5 may optionally comprise additions or deletions (i.e., gaps) as compared to the reference sequence (which does not comprise additions or deletions) for optimal alignment of the two sequences. The percentage is calculated by determining the number of positions at which the identical residue (e.g., nucleic acid base or amino acid residue) occurs in both sequences to yield the number of matched positions, dividing the number of matched 10 positions by the total number of positions in the window of comparison and multiplying the result by 100 to yield the percentage of sequence identity. Percentage sequence identity can be calculated by the local homology algorithm of Smith & Waterman, (1981) Adv. Appl. Math. 2:482-485; by the homology alignment algorithm of Needleman & Wunsch, (1970) J. Mol. Biol. 48:443-445; or by computerized implementations of these algorithms (GAP & BESTFIT in the GCG Wisconsin Software Package, Genetics Computer Group) or by manual alignment and visual inspection. Percentage sequence identity when calculated using the programs GAP or BESTFIT is calculated using default gap weights. The BESTFIT program has two alignment variables, the gap creation penalty and the gap extension penalty, which can be modified to alter the stringency of a nucleotide and/or amino acid alignment produced by the program. Parameter values used in the percent identity determination were default values previously established for version 8.0 of BESTFIT (see Dayhoff, (1979) Atlas of Protein Sequence and Structure, National Biomedical Research Foundation, pp. 353 25 358).

Probe design: One of skill in the art will appreciate that an enormous number of array designs are suitable for the practice of this invention. In some preferred embodiments, a high density array may be used. The high density array will typically include a number of probes that specifically hybridize to the sequences of interest (see WO 99/32660 for methods of producing probes for a given gene or genes). In addition, in a preferred embodiment, the array will include one or more control probes. High density array chips of the invention include "test probes" as defined herein. Test probes could be oligonucleotides that range from about 5 to about 45 or 5 to about 5 500 nucleotides, more preferably from about 10 to about 40 nucleotides and most preferably from about 15 to about 40 nucleotides in length. In other particularly preferred embodiments, the probes are 20 or 25 nucleotides in length. In another preferred embodiment, test probes are double or single strand nucleic acid sequences, preferably DNA sequences. Nucleic acid sequences may be isolated or cloned from natural sources or amplified from natural sources using native nucleic acid as templates. These probes have sequences complementary to particular subsequences of the genes whose expression they are designed to detect. Thus, the test probes are capable of specifically hybridizing to the target nucleic acid they are to detect. In addition to test probes that bind the target nucleic acid(s) of interest, the high density array can contain a number of control probes. The control probes fall into three categories referred to herein as (1) normalization controls; (2) expression level controls; and (3) mismatch controls. Normalization controls are oligonucleotide or other nucleic acid probes that are complementary to labeled reference oligonucleotides or other nucleic acid sequences that are added to the nucleic acid sample to be screened. The signals obtained from the normalization controls after hybridization provide a control for variations in hybridization conditions, label intensity, "reading" efficiency and other factors that may cause the signal of a perfect hybridization to vary between arrays. In a preferred embodiment, signals (e.g., fluorescence intensity) read from all other probes in the array are divided by the signal (e.g., fluorescence intensity) from the control probes thereby normalizing the measurements. Virtually any probe may serve as a normalization control. However, it is recognized that hybridization efficiency varies with base composition and probe length. Preferred normalization probes are selected to reflect the average length of the other probes present in the array, however, they can be selected to cover a range of lengths. The normalization control(s) can also be selected to reflect the (average) base composition of the other probes in the array, however in a preferred embodiment, only one or a few probes are used and they are selected such that they hybridize well (i. e., no secondary structure) and do not match any target-specific probes. Expression level controls are probes that hybridize specifically with constitutively expressed genes in the biological sample. Virtually any constitutively expressed gene provides a suitable target for expression level controls. Typically expression level control probes have sequences complementary to subsequences of constitutively expressed "housekeeping genes" including, but not limited to the actin gene, the transferrin receptor gene, the GAPDH gene, and the like. Mismatch controls may also be provided for the probes to the target genes, for expression level controls or for normalization controls. Mismatch controls are oligonucleotide probes or other nucleic acid probes identical to their corresponding test or control probes except for the presence of one or more mismatched bases. A mismatched base is a base selected so that it is not complementary to the corresponding base in the target sequence to which the probe would otherwise specifically hybridize. One or more mismatches are selected such that under appropriate hybridization conditions (e.g., stringent conditions) the test or control probe would be expected to hybridize with its target sequence, but the mismatch probe would not hybridize (or would hybridize to a significantly lesser extent). Preferred mismatch probes contain a central mismatch. Thus, for example, where a probe is a twenty-mar, a corresponding mismatch probe will have the identical sequence except for a single base mismatch (e.g., substituting a G. C or T for an A) at any of positions six through fourteen (the central mismatch). Mismatch probes thus provide a control for non-specific binding or cross hybridization to a nucleic acid in the sample other than the target to which the probe is directed. Mismatch probes also indicate whether a hybridization is specific or not. For example, if the target is present the perfect match probes should be consistently brighter than the mismatch probes. In addition, if all central mismatches are present, the mismatch probes can be used to detect a mutation. The difference in intensity between the perfect match and the mismatch probe provides a good measure of the concentration of the hybridized material.

Nucleic Acid Samples: As is apparent to one of ordinary skill in the art, nucleic acid samples, which may be DNA and/or RNA, used in the methods and assays of the invention may be prepared by any available method or process. Methods of isolating total mRNA are well letdown to those of skill in the art. For example, methods of isolation and purification of nucleic acids are described in detail in Chapter 3 of Tijssen, (1993) Laboratory Techniques in Biochemistry and Molecular Biology: Hybridization With Nucleic Acid Probes, Elsevier Press. Such samples include RNA samples, but also include cDNA synthesized from a mRNA sample isolated from a cell or tissue of interest. Such samples also include DNA amplified from the cDNA, and RNA transcribed from the amplified DNA. One of skill in the art would appreciate that it is desirable to inhibit or destroy RNase present in homogenates before homogenates can be used. Biological samples may be of any biological tissue or fluid or cells from any organism as well as cells raised in. vitro, such as cell lines and tissue culture cells. Frequently, the sample will be a "clinical sample" which is a sample derived from a patient. Typical clinical samples include, but are not limited to, sputum, blood, blood cells (e.g., white cells, serum markers), tissue or fine needle biopsy samples, urine, peritoneal fluid, and pleural fluid, or cells therefrom. Bone changes can be also observed with non-invasive scanning techniques. Biological samples may also include sections of tissues, such as frozen sections or formalin fixed sections taken for histological purposes.

Forming High Density Arrays: Methods of forming high density arrays of oligonucleotides with a minimal 25 number of synthetic steps are known. The oligonucleotide analogue array can be synthesized on a solid substrate by a variety of methods, including, but not limited to, light- directed chemical coupling, and mechanically directed coupling (see U.S. Patent 5,143,854). in brief, the light-directed combinatorial synthesis of oligonucleotide arrays on a 30 glass surface proceeds using automated phosphoramidite chemistry and chip masking techniques. In one specific implementation, a glass surface is derivatized with a silane reagent containing a functional group, e.g. a hydroxyl or amine group blocked by a photolabile protecting group. Photolysis through a photolithogaphic mask is used selectively to expose functional groups which are then ready to react with incoming 5' photoprotected nucleoside phosphoramidites. The phosphoramidites react only with those sites which are illuminated (and thus exposed by removal of the photolabile blocking group). Thus, the phosphoramidites only add to those areas selectively exposed from the preceding step. These steps are repeated until the desired array of sequences have been synthesized on the solid surface. Combinatorial synthesis of different oligonucleotide analogues at different locations on the array is determined by the pattern of illumination during synthesis and the order of addition of coupling reagents. In addition to the foregoing, additional methods which can be used to generate an array of oligonucleotides on a single substrate are described in WO 93/09668. High density nucleic acid arrays can also be fabricated by depositing premade or natural nucleic acids in predetermined positions. Synthesized or natural nucleic acids are deposited on specific locations of a substrate by light directed targeting and oligonucleotide directed targeting. Another embodiment uses a dispenser that moves from region to region to deposit nucleic acids in specific spots.

Hvbridization: Nucleic acid hybridization simply involves contacting a probe and target nucleic acid under conditions where the probe and its complementary target can form stable hybrid duplexes through complementary base pairing (see WO 99/32660). The nucleic acids that do not form hybrid duplexes are then washed away leaving the hybridized nucleic acids to be detected, typically through detection of an attached detectable label. It is generally recognized that nucleic acids are denatured by increasing the temperature or decreasing the salt concentration of the buffer containing the nucleic acids. Under low stringency conditions (e.g. , low temperature and/or high salt) hybrid duplexes (e.g., DNA:DNA, RNA:RNA or RNA:DNA) will form even where the annealed sequences are not perfectly complementary. Thus specificity of hybridization is reduced at lower stringency. Conversely, at higher stringency (e.g., higher temperature and/or lower salt and/or in the presence of destabilizing reagents) successful hybridization tolerates fewer mismatches. One of skill in the art will appreciate that hybridization conditions may be selected to provide any degree of stringency. In a preferred embodiment, hybridization is performed at low stringency in this case in 6x SSPE-T at 37°C (0.005% Triton x-100) to ensure hybridization and then subsequent washes are performed at higher stringency 5 (e.g., 1 x SSPE-T at 37°C) to eliminate mismatched hybrid duplexes. Successive washes may be performed at increasingly higher stringency (e.g., down to as low as 0.25x SSPET at 37°C to 50°C) until a desired level of hybridization specificity is obtained. Stringency can also be increased by addition of destabilizing agents such as formamide. Hybridization specificity may be evaluated by comparison of hybridization to the test probes with hybridization to the various controls that can be present (e.g., expression level control, normalization control, mismatch controls, etc.). In general, there is a trade-off between hybridization specificity (stringency) and signal intensity. Thus, in a preferred embodiment, the wash is performed at the highest stringency that produces consistent results and that provides a signal intensity greater than approximately 10% of the background intensity. Thus, in a preferred embodiment, the hybridized array may be washed at successively higher stringency solutions and read between each wash. Analysis of the data sets thus produced will reveal a wash stringency above which the hybridization pattern is not appreciably altered and which provides adequate signal for the particular oligonucleotide probes of interest.

Signal Detection: The hybridized nucleic acids are typically detected by detecting one or more labels attached to the sample nucleic acids. The labels may be incorporated by any of a number of means well known to those of skill in the art (see WO 99/32660).

Databases: The present invention includes relational databases containing sequence information, for instance, for the genes and members of the gene families of Table 1 or 2 as well as gene expression information in various tissue samples saved on computer readable medium and/or a user interface. Databases may also contain information associated with a given sequence or tissue sample such as descriptive information about the gene associated with the sequence information, or descriptive information concerning the clinical status of the tissue sample, or the patient from which the sample was derived. The database may be designed to include different parts, for instance a sequence database and a gene expression database. Methods for the configuration and construction 5 of such databases are widely available, for instance, see U.S. Patent 5,953,727, which is herein incorporated by reference in its entirety. The databases of the invention may be linked to an outside or external database. In a preferred embodiment, the external database is GenBank and the associated databases maintained by the National Center for Biotechnology Information (NCBI). Any appropriate computer platform may be used to perform the necessary comparisons between sequence information, gene expression information and any other information in the database or provided as an input. For example, a large number of computer workstations are available from a variety of manufacturers, such has those available from Silicon Graphics. Client/server environments, database servers and networks are also widely available and appropriate platforms for the databases of the invention. The databases of the invention may be used to produce, among other things, electronic Northerns that allow the user to determine the cell type or tissue in which a given gene is expressed and to allow determination of the abundance or expression level of a given gene in a particular tissue or cell. The databases of the invention may also be used to present information identifying the expression level in a sample of a set of genes comprising one or more of the sequences of genes or members of the gene families of Table 1 or 2, comprising comparing the expression level of at least one gene or member of a gene family of Table 1 or 2 in the sample to the level of expression of the gene in the database. Such methods may also be used in the drug or agent screening assays as described below.

Diagnostic Uses for the Differentiation Markers: As described above, the genes and gene expression information provided in Table 1 or 2 may be used as diagnostic markers for the prediction or identification of the differentiation state of a sample comprising precursor stem cells. For instance, a tissue sample may be assayed by any of the methods described above, and the expression levels from a gene or member of a gene family from Table 1 or 2 may be compared to the expression levels found in un- differentiated precursor stem cells and/or precursor stem cells induced to differentiate into osteoblasts and/or precursor stem cells induced to differentiate into a cell type other than an osteoblast and/or osteoblasts. The comparison of expression data, as well as available sequence or other information may be done by researcher or diagnostician or may be done with the aid of a computer and databases as described above. Such methods may be used to diagnose or identify conditions 15 characterized by abnormal bone deposition, re-absorption and/or abnormal rates of osteoblast differentiation. Those skilled in the art will appreciate that a wide variety of conditions are associated with abnormal bone deposition or loss. Such conditions include, but are not limited to, osteoporosis, osteopenia, osteodystrophy, and various other osteopathic conditions. The methods of the present invention will be particularly useful in diagnosing or monitoring the treatment of bone diseases, including, but not limited to postmenopausal osteoporosis (PMO), glucocorticoid-induced osteoporosis (GIO), male osteoporosis, acute bone loss, tumor-induced osteolysis, Paget's disease, and osteogenesis imperfecta.

Agents which modulate the expression of one or more the genes or gene families identified in Table 1 or 2 and/or modulate the activity of one or more of the proteins encoded by one or more of the genes or members of a gene family identified in Table 1 or 2 will be useful in treatment of the conditions. Some preferred embodiments, the present invention may be used to diagnose and/or monitor the treatment of drug-induced abnormalities in bone formation or loss. For example, at present a combination of cyclosporine with prednisone is given to patients who have received an organ transplant in order to suppress tissue rejection. The combination causes rapid bone loss in a manner different than that observed with prednisone alone (such as elevated level of serum osteocalcin and 1,25(OH)2-Vitamin D in patients treated with cyclosporine but not in patients treated with prednisone). Other drugs are also known to effect bone formation or loss. The anticonvulsant drugs diphenylhydantoin, phenobarbital and carbamazepine, and combination of these drugs, cause alterations in calcium metabolism. A decrease in bone density is observed in patients taking anticonvulsant drugs. Although heparin is an effective therapy for thromboembolic disorders, increased incidences of osteoporotic fractures have been reported in patients with heparin therapy hence the present invention will be useful to monitor patients undergoing heparin treatment. Other embodiments of the present invention allow the diagnosis and/or monitoring of the treatment of other conditions that involve altered bone metabolism. For example, idiopathic juvenile osteoporosis (IJO) is a generalized decrease in mineralized bone in the absence of rickets or excessive bone resorption and typically occurs in children before the onset of puberty. In addition, thyroid diseases have been linked bone loss. A decrease in bone mass has been shown in patients with thyrotoxicosis causing these individuals to be at increased risk of having fractures. These individuals also sustain fractures at an earlier age than individuals who have never been thyrotoxic. Other conditions in which the present invention will be useful include multiple myeloma and leukemia. Nearly 60% of patients with multiple myelomas have bone fractures with focal and lytic bone lesions and osteosclereotic bone lesions. Leukemia may also be associated with diffuse osteopenia and vertebral fracture in patients with acute lymphoblastic leukemia. Another situation in which the present invention will be useful is the diagnosis and/or monitoring of the treatment of skeletal disease linked to breast cancer. Breast cancer frequently metastasizes to the skeleton and about 70% of patients with advanced cancer develop symptomatic skeletal disease. Moreover, the anticancer treatments presently in use have been shown to lead to early menopause and bone loss when given to premenopausal women. The present invention will be useful in diagnosing and/or monitoring the treatment of chronic anemia associated with abnormal bone formation or loss. Homozygous beta-thalassemia is usually described as an example of chronic anemia predisposing to osteoporosis. Patients with thalassemia have expansion of bone marrow space with thinning of the adjacent trabeculae. The present invention will be useful in diagnosing and/or monitoring the treatment of mastocytosis. Skeletal symptoms (osteopenia and vertebral fracture) are present in 60 to 75% of the patients with systemic mast cell disease. Other conditions in which the present invention will find application are: Fanconi syndrome where osteomalacia is a common feature; fibrous dysplasia, McCune-Albright syndrome refers to patients with fibrous dysplasia with a sporadic, developmental disorder characterized by a unifocal or multifocal expanding fibrous lesion of bone forming mesenchyme that often results in pain, fracture or deformity, osteogenesis imperfecta (OI, also called brittle bone disease) is associated with recurrent fractures and skeletal deformity, various skeletal dysplasias, i.e., osteochondroplasia which is characterized by abnormal development of cartilage and/or bone and other diseases such as gingival diseases such as gingivitis and periodontitis, Paget's disease, hypercalcemia of malignancy, e.g. tumour-induced hypercalcemia and metabolic bone disease, achodroplasia, mucopolysacchaidoses, dysostosis and ischemic bone diseases. The present invention will be particularly useful by providing one or markers which may be used as markers of bone turnover to determine osteoporosis. The present invention may also be used in in vitro assays or treatments as a marker of osteoblast differentiation and/or proliferation.

The agents of the present invention may be used for a variety of purposes. In a preferred embodiment, they may be used in fracture repair of all types, i.e., non-union fractures, spinal fusion, accelerated healing of all types fractures from minor greenstick or compression fractures to comminuted, complicated fractures. Both local administrations to these fractures as well as parenteral administration which increases cartilage and bone formation, increases bone mass, and increases bone strength rapidly may be used. Another preferred embodiment of the present invention is the use of bone formation modulating agents in periodontal disease and/or for increasing bone around teeth.

Use of the Differentiation Markers for Monitoring Disease Progression: As described above, the genes and gene expression information provided in Table 1 or 2 may also be used as markers for the monitoring of disease progression, such as osteoporosis. For instance, a tissue sample may be assayed by any of the methods described above, and the expression levels for a gene or member of a gene family from Table 1 or 2 may be compared to the expression levels found in undifferentiated precursor stem cells and/or precursor stem cells induced to differentiate into osteoblasts and/or precursor stem cells induced to differentiate into a cell type other than an osteoblast and/or osteoblasts. The comparison of the expression data, as well as available sequence or other information may be done by researcher or diagnostician or may be done with the aid of a computer and databases as described above. The markers of the invention may also be used to Pack or predict the progress or efficacy of a treatment regime in a patient. For instance, a patient's progress or response to a give drug may be monitored by creating a gene expression profile from a tissue or cell sample after treatment or administration of the drug. The gene expression profile may then be compared to a gene expression profile prepared from undifferentiated precursor stem cells and/or precursor stem cells induced to differentiate into osteoblasts and/or precursor stem cells induced to differentiate into a cell type other than an osteoblast and/or osteoblasts and/or from tissue or cells from the same patient before treatment. The gene expression profile may be made from at least one gene, preferably more than one gene, and most preferably all or nearly all of the genes in Table 1 or 2.

Use of the Differentiation Markers for Drug Screening: According to the present invention, the genes identified in Table 1 or 2 may be used as markers to evaluate the effects of a candidate drug or agent on a cell. A candidate drug or agent can be screened for the ability to stimulate the transcription or expression of a given marker or markers or to down- regulate or counteract the transcription or expression of a marker or markers. For instance, agents that modulate, induce or inhibit gene expression in a sample to that which resembles a gene expression profile in an osteoblast differentiated cell population may be screened for the ability to modulate the differentiation process, bone depositions, etc. According to the present invention one can also compare the specificity of a drug effect by looking at the number of markers which the drug has and comparing them. More specific drugs will have less transcriptional targets. Similar sets of markers identified for two drugs indicates a similarity of effects. Assays to monitor the expression of a marker or markers as defined in Table 1 or 2 may utilize any available means of monitoring for changes in the expression level of the nucleic acids of the invention. As used herein, an agent is said to modulate the expression of a nucleic acid of the invention if it is capable of up- or down-regulating expression of the nucleic acid in a cell. In one assay format, gene chips containing probes to one or more genes or members of a gene family from Table 1 or 2 may be used to directly monitor or detect changes in gene expression in the treated or exposed cell as described in more detail above. In another format, cell lines that contain reporter gene fusions between the open reading frame and/or 5' - 3' regulatory regions of a gene or member of a gene family in Table 1 or 2 and any assayable fusion partner may be prepared. Numerous assayable fusion partners are known and readily available including the firefly luciferase gene and the gene encoding chloramphenicol acetyltransferase (Slam et al., (1990) Anal. Biochem. 15 188:245-254). Cell lines containing the reporter gene fusions are then exposed to the agent to be tested under appropriate conditions and time. Differential expression of the reporter gene between samples exposed to the agent and control samples identifies agents which modulate the expression of the nucleic acid. Additional assay formats may be used to monitor the ability of the agent to modulate the expression of a gene or member of a gene family identified in Table 1 or 2. For instance, as described above, mRNA expression may be monitored directly by hybridization of probes to the nucleic acids of the invention. Cell lines are exposed to the agent to be tested under appropriate conditions and time and total RNA or mRNA is isolated by standard procedures such those disclosed in Sambrook et al., (1989) 25 Molecular Cloning: A Laboratory Manual, Cold Spring Harbor Laboratory Press. In another assay format, cells or cell lines are first identified which express the gene products of the invention physiologically. Cells and/or cell lines so identified would be expected to comprise the necessary cellular machinery such that the fidelity of modulation of the transcriptional apparatus is maintained with regard to exogenous contact of agent with appropriate surface transduction mechanisms and/or the cytosolic cascades. Such cell lines may be, but are not required to be, bone marrow derived. Further, such cells or cell lines may be transduced or transfected with an expression vehicle (e.g., a plasmid or viral vector) construct comprising an operable non-translated 5'-promoter containing end of the structural gene encoding the instant gene products fused to one or more antigenic fragments, which are peculiar to the instant gene products, wherein said fragments are under the transcriptional control of said promoter and are expressed as polypeptides whose molecular weight can be distinguished from the naturally occurring polypeptides or may further comprise an immunologically distinct tag or some other detectable marker or tag. Such a process is well known in the art (see Sambrook et al., (1989) Molecular Cloning: A Laboratory Manual, Cold Spring Harbor Laboratory Press). Cells or cell lines transduced or transfected as outlined above are then contacted with agents under appropriate conditions; for example, the agent comprises a pharmaceutically acceptable excipient and is contacted with cells comprised in an aqueous physiological buffer such as phosphate buffered saline (PBS) at physiological pH, Eagles balanced salt solution (BSS) at physiological pH, PBS or BSS comprising serum or conditioned media comprising PBS or BSS and/or serum incubated at 37°C. Said conditions may be modulated as deemed necessary by one of skill in the art. Subsequent to contacting the cells with the agent, said cells are disrupted and the polypeptides of the lysate are fractionated such that a polypeptide fraction is pooled and contacted with an antibody to be further processed by immunological assay (e.g., ELISA, immunoprecipitation or Western blot). The pool of proteins isolated from the "agent contacted" sample is then compared with a control sample Where only the excipient is contacted with the cells and an increase or decrease in the immunologically generated signal from the "agent- contacted" sample compared to the control is used to distinguish the effectiveness of the agent. Another embodiment of the present invention provides methods for identifying agents that modulate the levels or at least one activity of a protein(s) encoded by the genes in Table 1 or 2. Such methods or assays may utilize any means of monitoring or detecting the desired activity. In one format, the relative amounts of a protein of the invention between a cell population that has been exposed to the agent to be tested compared to an un-exposed control cell population may be assayed. In this format, probes such as specific antibodies are used to monitor the differential expression of the protein in the different cell populations. Cell lines or populations are exposed to the agent to be tested under appropriate conditions and time. Cellular lysates may be prepared from the exposed cell 5 line or population and a control, unexposed cell line or population. The cellular lysates are then analyzed with the probe, such as a specific antibody. Agents that are assayed in the above methods can be randomly selected or rationally selected or designed. As used herein, an agent is said to be randomly selected when the agent is chosen randomly without considering the specific sequences involved in the association of the a protein of the invention alone or with its associated substrates, binding partners, etc. An example of randomly selected agents is the use a chemical library or a peptide combinatorial library, or a growth broth of an organism. As used herein, an agent is said to be rationally selected or designed when the agent is chosen on a nonrandom basis which takes into account the sequence of the target site and/or its conformation in connection with the agent's action. Agents can be rationally selected or rationally designed by utilizing the peptide sequences that make up these sites. For example, a rationally selected peptide agent can be a peptide whose amino acid sequence is identical to or a derivative of any functional consensus site. The agents of the present invention can be, as examples, peptides, small molecules, vitamin derivatives, as well as carbohydrates. Dominant negative proteins, DNA encoding these proteins, antibodies to these proteins, peptide fragments of these proteins or mimics of these proteins may be introduced into cells to affect function.

"Mimic" used herein refers to the modification of a region or several regions of a peptide molecule to provide a structure chemically different from the parent peptide but topographically and functionally similar to the parent peptide (see Meyers, (1995) Molecular Biology and Biotechnology, VCH Publishers, 659-664). A skilled artisan can readily recognize that there is no limit as to the structural nature of the agents of the present invention. Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

### Examples

### Methods:

*Cell culture and stimulation:* MC3T3-E1 cell line was a kind gift of T. Kokkubo (Novartis, Japan) (Sudo et al., 1983, J Cell Biol; 96:191-198). The MC3T3-1 b clone was generated by stable transfection of Cbfa1 DNA response element OSE2 upstream of the reporter gene Luciferase into MC3T3-E1 cell line mentioned above. The MC3T3-1 b cell clone was selected on the basis of activation of the Luciferase gene by the osteogenic stimulus, comprising 200 ng/ml BMP-2 (bone morphogenetic protein - 2), 50 µM ascorbic acid and 10 mM β-glycerophosphate. The cells were grown in α-MEM with 10% fetal calf serum (FCS, Gibco), 1% penicilin/streptomycin (Pen/Strep) and 1% L-Glutamine (L-Glu) in T175 flasks (40 ml/flask). The stimulation was done in the same medium.

For RNA isolation, cells were plated on 6 cm dishes for (3x10^{5 cells}/dish in 5 ml medium), for alkaline phosphatase staining on 48-well plates (1x10⁴ cells/well in 1 ml medium) and for Alizarin red S staining on 12-well plates (5x10⁴ cells/well in 3 ml medium). The cells were grown to confluence for 3 days at 37°C / 5% CO₂, and then stimulated with 10 mM β-glycerophosphate (GP, Sigma), 50 µM ascorbic acid (AA, Wako) and 200 ng/ml BMP-2 (Nico Cerletti, Novartis). The control cells were stimulated with 10 mM β-glycerophosphate. For Alizarin Red S staining, the medium together with stimulators was changed twice weekly. *RNA isolation:* RNA was extracted from non-stimulated confluent cells (day 0), and from the cells treated with GP/AA/BMP-2 or GP alone for 1 and 3 days. The cells were harvested in the Lysis Buffer containing guanidinium-isothiocyanate. The total RNA was extracted, treated with DNAse I and purified according to manufacturer's protocol (RNeasy mini kit, QIAGEN; Basel, Switzerland).

*Alkaline phosphatase staining:* Three days after stimulation, the cells were washed twice with PBS, fixed with formalin /methanol / H₂O (1:1:1.5), 0.5 ml/well, incubated 15 min at room temperature, and washed 3 times with water. For staining, one FAST BCIP/NBT tablet from Sigma (5-bromo-4-chloro-3-indolyl phosphate / nitro blue tetrazolium, alkaline phosphatase substrate) was dissolved in 10 ml of water. To the fixed cultures, 0.5 ml of substrate solution was added and incubated for 15 min at room temperature. After staining, the cultures were washed 3 times with water and air-dried.

*Alizarin red S staining for mineralization:* Fourteen days after stimulation, the cells were washed twice in PBS, fixed with formalin /methanol / H₂O (1:1:1.5), 0.5 ml/well, 15 minutes at room temperature, and 3 times washed with water. Saturated Alizarin Red S solution was filtered, 1.5 ml was added per well, and incubated for 15 min at room temperature. The cells were washed 4-5 times with water and air-dried.

*Quantitative radioactive RT-PCR (qrRT-PCR):* qrRT-PCR was used to analyze expression profiles of the marker genes prior to microarray analysis, and to confirm microarray data for some selected genes.First strand complementary DNA was synthesized according to the standard protocol. Briefly, 10 units of RNAse inhibitor (ROCHE Molecular Diagnostics) and 100 µg of random hexanucleotides (Amersham Pharmacia Biotech) were added to 1 µg of DNAse I-treated total cellular RNA. Samples were denaturated for 5 min at 65°C and chilled on ice. Then, samples were filled to 20 µl with a nuclease-free solution containing another 10 units of RNAse inhibitor, 2.5 mM of each dNTP, 50 mM Tris-HCl pH 8.3, 60 mM KCI, 10 mM MgCl₂ and 1 mM DTT. 20 units of Avian Myeloblastosis Virus reverse transcriptase (Stratagene) was added (cDNA) or not (RT-) to each sample. Reverse transcription reaction was performed for 2 h at 42°C, and stopped by incubation for 5 min at 95°C. Samples were diluted 5-fold with nuclease-free water and stored at -80°C until use. One µl of cDNA or RT-sample was used as a PCR template. PCR reaction was performed in a final volume of 25 µl, containing 100 µM of each dNTP, 1 µCi of α [³²P]-dATP, 1 µM of each primer and 1.25 units of "Hot start" thermostable DNA polymerase and corresponding reaction buffer (FastStart Taq, ROCHE Molecular Diagnostics). For PCR analysis of PTHR, DIx2, RAMP1, JunB, Fra-1, and Wnt6, the reaction mix contained in addition 5 % glycerol. The amplification protocol was the following: initial step of 5 min at 94°C, 12-33 cycles of denaturation at 94° C for 1 min, annealing at 57/60° C (all genes at 57° C, except PTHR, DIx2, RAMP1, JunB, Fra-1, at 60° C) for 1 min, and extension at 72° C for 1 min 20 s. The amplification was terminated with the final incubation step at 72° C for 10 min. Aliquots of PCR products were mixed with a loading buffer (final concentrations: 5 % glycerol, 10 mM EDTA, 0.01 % SDS, 0.025 % xylene cyanol and bromophenol blue dyes) and analyzed on 8 % polyacrylamide gels. The gels were vacuum-dried, exposed to phosphor-storage screens (Molecular Dynamics) and imaged by Phosphorimager (Molecular Dynamics). The signals on images were quantified by the ImageQuant software (Molecular Dynamics). For each gene analyzed, a cycle curve experiment was performed and the optimal number of PCR cycles for the quantitative analysis was chosen within the linear range of amplification. The primers (forward and reverse, given in the 5' to 3' orientation) and the number of cycles used in PCR are listed below.

| Gene: | Primers: |
|---|---|
| Alkaline phosphatase (ALP) | |
| | CCCAAAGGCTTCTTCTTGC and |
| | GCCTGGTAGTTGTTGTGAG, 30 cycles |
| Msx2: | |
| | CGCCTCGGTCAAGTCGGAA and |
| | GCCCGCTCTGCTAGTGACA, 31 cycles |
| Parathyroid hormone receptor (PTHR): | |
| | ACCCCGAGTCTAAAGAGAAC and |
| | GCCTTTGTGGTTGAAGTCAT, 28 cycles |
| Osteocalcin (OCN): | |
| | GGGCAATAAGGTAGTGAACAG and |
| | GCAGCACAGGTCCTAAATAGT, 28 cycles |
| Cbfa1(α/m and ε): | |
| | ATGCTTCATTCGCCTCAC and |
| | CTCACGTCGCTCATCTTG, 29 cycles |
| Osteopontin (OPN): | |
| | CACAAGCAGACACTTTCACTC and |
| | GAATGCTCAAGTCTGTGTGTT , 23 cycles |
| Osteonectin (ONC): | |
| | CCCTGCCAGAACCATCATTG and |
| | TTGCATGGTCCGATGTAGTC, 23 cycles |
| Collagen 1α I (Col1a): | |
| | CCCTGCCTGCTTCGTGTAAA and |
| | CCAAAGTCCATGTGAAATTATC, 22 cycles |
| 18S ribosomal subunit RNA (18S rRNA): | |
| | CCTGGATACCGCAGCTAGGA and |
| | GCGGCGCAATACGAATGCCCC, 12 cycles |
| Glyceraldehyde 3-phosphate dehydrogenase (GAPDH): | |
| | CTGCACCACCAACTGCTTAG and |
| | AGATCCACGACGGACACATT, 19 cycles |
| SMAD1: | |
| | TGCTGGTGGATGGTTTCACA and |
| | TGTCGCCTGGTGTTTTCAATA , 29 cycles |
| SMAD6: | |
| | GCAACCCCTACCACTTCAG and |
| | GCCTCGGTTTCAGTGTAAGA, 28 cycles |
| JunB: | |
| | CAGCCTTTCTATCACGACGA and |
| | GGTGGGTTTCAGGAGTTTGT, 31 cycles |
| Id2: | |
| | CCGATGAGTCTGCTCTACAAand |
| | CCGTGTTCAGGGTGGTCAG, 27 cycles |
| DIx2: | |
| | AAACCACGCACCATCTACTC and |
| | TCGCCGCTTTTCCACATCTT, 31 cycles |
| Fra-1: | |
| | ACCGCCCAGCAGCAGAAGT and |
| | AGGTCGGGGATAGCCAGTG, 30 cycles |
| Wnt6: | |
| | CCGACGCTGGAACTGCTC and |
| | ATGGAACAGGCTTGAGTGAC, 33 cycles |
| TCF7: | |
| | ACTCTGCCTTCAATCTGCTC and |
| | GGGTGTGGACTGCTGAAATG, 27 cycles |
| Low density lipoprotein receptor-related protein 5 (LRP5): | |
| | GCCAGTGTGTCCTCATCAAG and |
| | ACGCTGGCAGACAAAGTAGA, 25 cycles |
| Transforming growth factor β1 (TGFβ1): | |
| | CCAAAGACATCTCACACAGTAand |
| | TGCCGTACAACTCCAGTGAC, 27 cycles |
| Transforming growth factor β3 (TGFβ3): | |
| | CACCGCTGAATGGCTGTCT and |
| | CATTGGGCTGAAAGGTGTGA, 26 cycles |
| TIEG: | |
| | TTCAGCAGCAAGGGTCACTC and |
| | GACAGGCAAACTTCTTCTCAC, 28 cycles |
| Hey1: | |
| | GCCGACGAGACCGAATCAAT and |
| | GCTGGGATGCGTAGTTGTTG, 30 cycles |
| FK506-binding protein 5 (FKBP5): | |
| | GCTGCCATCGTGAAAGAGAAand |
| | TCCACGGCTTTGTTGTACTC, 29 cycles |
| Receptor activity-modifying protein 1 (RAMP1): | |
| | TCTGGCTGCTGCTGGCTCA and |
| | TTTCCCCAGTCACACCATAG, 31 cycles |
| HIx: | |
| | CGGGACAGTTCTTCGCATCT and |
| | TTCGGTCTGGCTTGGTCAC, 28 cycles |

*Gene expression determination using high-density oligonucleotide microarrays:* Total RNA form each sample (treatment: non-stimulated, day 0; GP/AA/BMP-2, day 1 and 3; GP day 1 and 3) was extracted and analyzed on 5 oligonucleotide microarrays. The microarray hybridization was performed using the Affymetrix protocols (REF): Affymetrix GeneChip^{®} Murine Genome U74Av2 arrays were used, which consist of coated glass slides with a series of oligonucleotide probes synthesized *in situ*. These arrays contain probes for approximately 9,400 genes (∼5,700 functionally characterized genes and ∼ 3,700 EST clusters). Biotin-labeled cRNA probes were generated from each sample to be analyzed, starting form 5 µg of DNAse I-treated total cellular RNA, prepared as described above. The cRNA probes were individually hybridized on the arrays and the signals were detected according to the manufacturer's instructions (Affymetrix, Santa Clara, CA, USA).

Analysis of gene expression on microarrays: The hybridization data were analyzed by the software provided by Affymetrix (MAS4.0), by Novartis Pharmacogenetics Network (NPGN) software and Expressionist 3.0 (GeneData, Basel, Switzerland). Genes were considered as expressed if they were classified as P (present), and not as M (marginal) or A (absent) at least in one sample. The cut-off for classification as present was hybridization signal of 20; all lower numbers were clamped to 20. All genes presented in detail were detected with gene-specific probes, and not with probe sets that recognize the gene families. Genes were selected as regulated by GP/AA/BMP-2 treatment if their expression deviated more than 2-fold from the corresponding, time-matched control.

### Example 1: Osteoblastic differentiation of MC3T3-1b cell line: cellular and molecular characterization

The instability of osteoblastic phenotype in cell lines in culture is a known phenomenon, leading to divergent properties of an apparently identical cell line. Murine pre-osteoblastic MC3T3-E1 cell line is a very good model of osteoblast differentiation, reproducing in vitro a sequence of several events occurring in vivo (Quarles et al., 1992, J. Bone Miner Res; 7:683-692). In order to obtain a cell line that differentiates in vitro in a fast and efficacious manner, we transfected MC3T3-E1 parental cells with a Cbfa1-dependent reporter gene and selected a clone of MC3T3-E1 cells that responds well to osteogenic stimulus (10 mM GP, 50 µM AA and 200 ng/ml BMP-2). This clone was named MC3T3-1 b and characterized with respect to cellular phenotype and molecular markers of differentiation. In response to osteogenic stimulus, MC3T3-1 b cell line showed a strong increase in alkaline phosphatase activity at day 3, as documented by cytochemical staining. Furthermore, this cell line produced bone nodules in culture and mineralized them by day 14, as shown by Alizarin Red S staining. After several more days, the number of nodules increased greatly, covering more than 50 % of well area. This result showed that in this cell line a whole differentiation process is reproduced within 2 weeks, which is very fast and efficient. Previously, we and others noticed that with other MC3T3-E1 clones it is necessary to maintain cultures for 3-5 weeks to obtain mineralized bone nodules, and the whole process was often inefficient (Quarles et al., 1992, as above). Thus, MC3T3-1b cells exhibit expected cellular properties for the study of osteoblast differentiation process.

Next, we analyzed messenger ribonucleic acid (mRNA) expression of a number of molecular markers of osteoblast differentiation by radioactive quantitative reverse transcription-polymerase chain reaction (rqRT-PCR). MC3T3-1 b cells were stimulated with osteogenic stimulus for 1 and 3 days and compared with non-stimulated time-matched controls. Eight markers of osteoblast differentiation were analyzed: alkaline phosphatase (ALP), transcription factors Msx2 and Cbfa1, parathyroid hormone receptor (PTHR), and extracellular matrix proteins osteocalcin (OCN), osteopontin (OPN), osteonectin (ONC) and collagen Iα1 (Col Iα1). The optimal primers were designed and quantitative conditions were optimized in cycle curve rqRT-PCR experiments for each marker gene (see Methods above). In response to the osteogenic stimulus, mRNA levels of five osteoblast markers were up-regulated in MC3T3-1 b cells. ALP was strongly induced already at day 1 with even higher increase at day 3. Msx2 was transiently up-regulated at day 1 and Cbfa1 was induced at day 3. PTHR and OCN, late markers of osteoblast differentiation, were strongly induced at day 3.

For three osteoblast markers, OPN, ONC and Col Iα1, high levels of expression were detected already in non-stimulated conditions and they did not change upon treatment. As MC3T3-1b cells are already committed to the osteoblast phenotype, high level of these extracellular matrix proteins may be expected. As a normalization control, mRNA levels of two housekeeping genes analyzed: 18S ribosomal RNA (18SrRNA) and glyceraldehyde-3 phosphate dehydrogenase (GAPDH). Their expression was similar in all conditions.

### Example 2: Gene expression profiling by DNA microarray analysis: comparison of osteoblast marker expression by rqRT-PCR and microarrays:

After selection of an appropriate system of in vitro osteoblast differentiation, we set out to perform genome-wide analyses of gene expression during osteoblast differentiation. MC3T3-1 b cell line was stimulated with osteogenic stimulus, total RNA was extracted at days 0, 1 and 3 and analyzed by Affymetrix GeneChip microarray U74. This oligonucleotide array represents about 10,000 genes, comprising genes with known function and ESTs.

As a validation step in the microarray data analysis, hybridization values obtained for osteoblast marker genes were compared with the data obtained by rqRT-PCR. The GeneChip contained oligonucleotide probes for seven of eight osteoblast markers and for one of two housekeeping genes analysed by rqRT-PCR. For these seven markers and one housekeeping gene, the same trend and a similar fold regulation was observed with two different methods. Fold up-regulation differed mostly for the genes that were up-regulated from the background to very high levels (PTHR, OCN). This is expected due to the inaccuracy of background measurement. There was a tendency to obtain smaller fold changes by microarray analysis. Strong and reliable hybridization microarray signals were obtained even for the genes with very low level of expression, indicating a high sensitivity of microarray detection in our experiments. We concluded that microarray data obtained can provide reliable information about gene expression profiles, even for the very low expressed genes like transcription factors.

### Regulated expression of 382 known genes and 283 ESTs

To get an insight into transcriptional events involved in the osteoblast differentiation process, we investigated genes whose expression levels changed upon treatment with osteogenic stimulus. The levels of gene expression in treated samples was compared with time-matched non-stimulated controls. A 2-fold up- or down-regulation was taken as a selection threshold. We detected 382 genes with known function and 283 ESTs, whose expression changed at least 2-fold at one or both time points post-stimulation (day 1 or 3 or both). In further analyses, we focused on the genes with known function.

The functional analysis of 382 regulated genes led us to define ten large groups of regulated genes: 1) Matrix Proteins and Adhesion (37); 2) Growth Factors (26); 3) Receptors (31); 4) Cytoskeleton (12); 5) Signaling (25); 6) Kinases (16); 7) Transcription Factors (51); 8) Cell cycle and apoptosis (20); 9) DNA replication (22); and 10) Others (142) (Table 1). In Table 1, within each of these groups, the genes were sorted from high to low degree of regulation, including both days 1 and 3. Most of these genes were not analyzed further and will not be discussed in detail, but they provided a complete picture of transcriptional events during osteoblast differentiation in our model system.

A large number of regulated genes (37) belonged to the group related to Matrix Proteins and Adhesion (Table 1). This is consistent with osteoblasts being adherent cells responsible for production of large amounts of bone matrix. Down-regulation of many adhesion molecules and up-regulation of others suggested that osteoblasts change their adhesion properties during differentiation. Most prominent up-regulated genes were bone sialoprotein, osteocalcin, osteomodulin and keratoepithelin (3.5-13-fold). Among down-regulated genes were cadherin 11 (osteoblast-specific cadherin), osteoglycin, laminin, thrombospondin, vascular cell adhesion molecule-1 (VCAM-1) and matrix metalloproteinase-13 (MMP-13). This pattern fits the expected changes, but also reveals some new aspects, especially among down-regulated genes.

The Growth Factors group contained genes expected to be regulated in osteoblasts, but also some novel genes (Table 1). Some of the expected genes are the known modulators of osteoblast proliferation: PDGFα, VEGF, FGF, IGF binding proteins, PTH-RP (Canalis and Rydziel, 1996, Chapter 44, 619-626, Academic Press Inc; Hurley and Florkiewicz, 1996, Chapter 45, 627-645, Academic Press Inc.; Conover, 1996, Chapter 43, 607-618, Principles of Bone Biology; Swarthout et al., 2002, Gene, 282:1-17). Some of the novel regulated genes were those of extracellular factors TGF-β and Wnt families (TGF-β1-3 and BMP-4, and Wnt1, 6, and 10a) and of their extracellular regulators (BMP inhibitor Gremlin). Although their connection to bone physiology is known (TGF-β, Kim and Ballock, 1993, J Cell Biochem. 77:169-78) or only recently suggested (Wnt, Gong et al., 2001, Cell, 107:513-523), their expression by osteoblasts and regulation during differentiation has not yet been reported. Wnt 10a is the most prominent stimulated gene, with 6.8-fold up-regulation, while Gremlin is the most prominently inhibited gene, with 0.12 relative to control. Interestingly, many members of the corresponding signaling pathways of TGF-β and Wnt were also regulated, as will be shown and discussed below. Furthermore, M-CSF, small inducible cytokine A7 and PTH-RP, all of which can stimulate differentiation or function of bone-resorbing osteoclasts, were down-regulated, suggesting reduced ability of differentiating osteoblasts to stimulate osteoclasts.

In the Receptors group, up-regulated genes included those coding for the receptors of many factors implicated in osteoblast differentiation or function: PTH receptor PTHR, LIF receptor LIFR, leptin receptor LEPR, prostaglandin F receptor PTGFR, FGF receptor FGFR2, nuclear orphan receptor RORα, urokinase receptor PLAUR, Neuropilin 1/VEGF165 receptor (Swarthout et al., 2002, Gene, 282:1-17; Malaval and Aubin, 2001, J Cell Biochem, 81(S36):63-70; Gordeladze et al., 2001, Curr Pharm Des, 7:275-290; Pilbeam et al., 1996, Chapter 51, 715-728, Academic Press Inc.; Wilkie, 1997 Hum Mol Genet, 6:1647-56; Meyer et al., 1999, PNAS USA, 97:9197-202; Allan et al., 1995Clin Orthop, 313:54-63; Deckers et al., 2000, Endocrinology, 141:1667-74) (Table 1). Some of the up-regulated receptors have not been reported previously to play a role in osteoblasts, such as ephrin receptors (EPHA2, EPHB2) and somatostatin receptors (SSTR4, SSTR2), both of which were up-regulated in our system 3-5-fold. The highest up-regulated receptor genes (>30-fold) were those for PTH receptor (PTHR) and a co-receptor for calcitonin receptor-like receptor RAMP1. While up-regulation of PTHR as an accepted osteoblast marker was expected, up-regulation of co-receptor RAMP1 was surprising. Interestingly, osteoprotegerin (OPG), a decoy receptor for RANKL, a main cytokine in osteoclastogenesis, was up-regulated, suggesting that differentiating osteoblasts have reduced ability to stimulate osteoclast formation. Down-regulated genes included secreted form of interleukin-4 receptor IL4R, vascular endothelial growth factor receptor VEGFR2, oncostatin receptor OSMR, platelet-derived growth factor receptor PDGFRα, low density lipoprotein receptor-related protein 5 LRP5, and prostaglandin E receptor 1 PTGER1 (Ura et al., 2000, Endocr J, 47:293-302; Hurley and Florkiewicz R, 1996, Chapter 45, 627-645, Principles of Bone Biology, Academic Press Inc.; Jay et al., 1996, Endocrinology, 137:1151-8; Canalis and Rydziel, 1996, Chapter 44, 619-626, Principles of Bone Biology, Academic Press Inc.; Pilbeam et al., 1996, Chapter 51, 715-728, Principles of Bone Biology, Academic Press Inc.). Most highly inhibited genes (0.11-0.13 of control) were those for the chemokine orphan receptor 1 (RDC1) and prostaglandin E2 receptor 1 (EP1). Reduced levels of mRNA for these receptors suggests that they perform a function in pre-osteoblasts and that their signals need to be reduced for osteoblast differentiation to proceed. This is easily understood for PDGFRα, which is not necessary any more after osteoblasts have completed their proliferation phase and started to differentiate. The Cytoskeleton, Signaling Molecules and Kinases groups are not mentioned in detail, because the connection of regulated genes with bone physiology is not clear at present. Nevertheless, some of these genes would also deserve a closer investigation in the future. We will only mention an interesting case of specific gene regulation within one family: the cytosolic protease calpain 6 was up-regulated 4.1-fold, while calpain 5 was expressed at 0.5-fold relative to the control.

The Transcription Factors group is the largest and contains many interesting regulated genes. Transcription factors are generally weakly expressed; however, we detected about 50 regulated transcription factors, all with a reliable signal intensity (Table 1). Furthermore, we analyzed the regulation of 10 transcription factor genes by rqRT-PCR and independently confirmed the regulation observed by microarray analysis (see below). A large number of regulated transcription factors suggested that their orchestrated regulation is key to the osteoblast differentiation. Many of them have not yet been linked to osteoblasts or bone, and for simplicity reasons we will focus on the up-regulated genes and those forming functionally-linked subgroups. They belong to the following structural subgroups: homeobox (Msx2, HOXC9, DIx2, and Hlx), Zn finger (Zac1, BRCA1, Egr2/Krox20, PML), and helix-loop-helix (Hey1, Id1-3, CLOCK). Functionally, they are on the signaling pathways of TGFβ family, Wnt extracellular factors and the Notch receptor. The genes belonging to these three signaling pathways are mentioned below.

The regulation of many genes involved in cell cycle and DNA replication is consistent with our observation that the cells, although confluent prior to stimulation, still continue to proliferate to a small degree up to 3 days post-stimulation with osteogenic stimulus. Finally, the genes in the group Others comprise those whose connection to bone physiology is presently unclear and which did not belong to any of the above mentioned large gene groups. In Table 1, we show the most interesting members of this group. In the rest of this study, we concentrated on the analysis of the genes belonging to the functionally most interesting groups.

### Example 3: Genes coding for proteins with established function in osteoblast differentiation and regulation of osteoclast formation

We first evaluated the expression of genes with an established function in osteoblast differentiation and osteoclast formation, two main processes in osteoblastic cells (Table 2). The identified genes were grouped into following categories: 1) BMP signaling pathway; 2) BMP target genes; 3) Osteoblast differentiation (including osteoblast markers); and 4) Stimulation of osteoclastogenesis. It should be noted that although the function of these genes has been linked to bone cells, for many of them, this type of regulation during osteoblast differentiation was not previously reported.

Major components of BMP-2 signaling pathway, SMAD molecules, were shown to be up-regulated 2-3-fold. The mRNA expression of SMAD 6 and 7, the inhibitory SMADs, was transiently up-regulated at day 1 (Table 2). This probably represents a feed-back mechanism for down-regulation of BMP signaling and is in agreement with a recent study (Locklin at al, 2001, J Bone Miner Res, 16:2192-204). SMAD1, BMP-specific SMAD, was up-regulated at day 3, showing that positive BMP signaling components are stimulated as well. Gremlin, the secreted BMP-2 antagonist, was strongly down-regulated by osteogenic stimulus at both days 1 and 3, suggesting further regulatory mechanism for stimulation of BMP-signaling. Some genes that were previously shown to be BMP-2 target genes in osteoblasts (Locklin at al, 2001, J Bone Miner Res, 16:2192-204) were also regulated in our study. JunB, AP-1 complex component that is involved in osteoblast differentiation (Jochum et al., 2001, Oncogene, 20:2401-12), was strongly up-regulated at both days 1 and 3. Id group of HLH transcriptional inhibitors (Lopez-Rovira et al., 2002, J Biol Chem, 277:3176-85). Id1, 2 and 3 were strongly up-regulated, while Id4 was down-regulated. The homeobox transcription factor DIx2, known to be important for craniofacial patterning and morphogenesis during-embryonic development (Xu et al., 2001, DNA Cell Biol, 20:359-65), was transiently up-regulated at day 1.

Other genes with function in osteoblasts were also regulated. Fra-1 (FosL-1), a transcription factor in focus of bone biology, was shown to be important both for bone formation in vivo and for osteoclast differentiation in vitro (Matsuo et al., 2000, Nat Genet, 24:184-7, Jochum et al., 2001, Oncogene, 20:2401-12). Its mechanism of action in osteoblasts in vitro has not been investigated. We detected a strong and transient up-regulation of Fra-1 (3.4-fold). Transcription factor Msx1 was shown to be down-regulated upon terminal differentiation of chondrocytes, muscle cells, and most recently, odontoblastic cells (Blin-Wakkach, 2001, PNAS USA, 98:7336-41 and references therein). We detected a strong down-regulation of Msx1 (0.25-fold relative to control). Leptin has been associated with bone physiology and the prevailing hypothesis suggests brain as its site of action (Ducy, 2000, Science, 289:1501-4). However, in-house studies have shown up-regulation of the leptin receptor during osteogenic differentiation of human mesenchymal cells. We confirmed the up-regulation of leptin receptor also in our MC3T3-1 b cell system (5-fold). Up-regulation of retinoic acid receptor related protein alpha (RORα) during osteogenic differentiation of human mesenchymal cells has also been shown in-house and we also detected 2.6-fold up-regulation. Most recent data suggested that a mutated low density lipoprotein receptor-related protein 5 (LRP5) is a high bone mass gene in humans, while other mutations lead to osteoporosis-pseudoglioma syndrome (Gong et al., 2001, Cell, 107:513-523; Little et al., 2002, Am J Hum Genet, 70:11-9; Boyden et al., 2002, N Engl J Med, 346:1513-21). In our system, LRP5 is down-regulated upon treatment with osteogenic stimulus at both days 1 and 3. BMP-4, a closely related gene to BMP-2 and strong osteogenic agent (Yamaguchi et al., 2000, Endocr Rev, 21:393-411), is also down-regulated.

Cells of osteoblastic lineage are stimulators of osteoclast formation, mainly via up-regulation of cytokines M-CSF and RANKL (reviewed in Takahashi et al., 1999, Biochem Biophys Res Commun, 256:449-55). Upon treatment with osteogenic stimulus, we detected up-regulation of OPG, an inhibitor of RANKL by 3.8-fold. M-CSF, PTH-RP, and small inducible cytokine A7, all of which can stimulate osteoclast formation and/or function are down-regulated. In agreement with that, the transcription factor Egr1, a negative regulator of M-CSF (Cenci et al., 2000, J Clin Invest, 105: 1279-87), was up-regulated by 3.6-fold. RANKL was not expressed by MC3T3-1 b cells before nor after stimulation with osteogenic stimulus. We conclude that MC3T3-1 b cells express and regulate molecules involved in osteoclast formation. The direction of regulation of these molecules suggests that the ability for the support of osteoclastogenesis is reduced during osteoblastic differentiation.

### Example 4: Activation of TGFβ signaling pathway

Three members of transforming growth factor β family (TGFβ) were regulated (TGFβ1, 4-fold up-regulation at days 1 and 3, TGFβ2, 2-fold down-regulation at day 3, and TGFβ3, 2-fold up-regulation at day 1 (Table 3). TGFβ family members are potent modulators of osteoblast differentiation process (reviewed in Kim and Ballock, 1993, Cellular and Molecular Biology of bone, Academic Press, 98-122), but their regulation by osteogenic stimulus containing BMP-2 has not been reported yet. Interestingly, two family members are up-regulated and one is down-regulated. Since MC3T3-1b cells are responsive to TGFβ, we investigated the expression of the components from TGFβ signaling pathway on microarrays. Type I TGFβ receptor was expressed, but not regulated, while signal for type II receptor was absent, suggesting a low expression level. SMAD2 probe was not present on the chip, while SMAD3 was not detected. From the Western blot data, we know that SMAD2 is present and phosphorylated in response to TGFβ-1. The inhibitory SMAD6 and 7 were transiently up-regulated, as mentioned above. The activation of TGFβ pathway by stimulation with the osteogenic stimulus was confirmed by regulation of several TGFβ target genes: transcriptional repressor TIEG (Hefferan et al., 2000, J Cell Biochem, 78:380-90); extracellular matrix protein tenascin C (Mackie et al., 1998, Bone 22: 301-7), adhesion molecule kerato-epithelin (Kim et al, 2000, J Cell Biochem, 77:169-78), and somatostatin receptor type 2 (SSTR2) (Puente et al., 2001, J Biol Chem, 276:13461-8). We conclude that stimulation of pre-osteoblasts with a mixture of GP, AA and BMP-2 induces production of TGFβ1 and 3, which are in turn capable of inducing TGFβ signaling cascade. It would be interesting to determine which component of the osteogenic stimulus leads to the induction of TGFβ1 and 3. These results shed a new light on the interplay between different growth factors in osteoblastic differentiation.

### Example 5: Activation of Wnt signaling pathway

Three members of Wnt family of secreted proteins was up-regulated: Wnt1, 2-fold, Wnt6, 4-fold, and Wnt10a, 5-fold, (Table 4). Wnt proteins are known to control multiple developmental processes (reviewed in Wodarz and Nusse, 1998, Annu Rev Cell Dev Biol, 14:59-88), including limb patterning and joint formation. Very recently they came into a focus of bone biology as it was shown that a co-receptor for Wnt, the low density lipoprotein related protein 5 (LRP5), was responsible for two striking human skeletal phenotypes, osteoporosis-pseudoglioma syndrome and high bone mass (Gong et al., 2001, Cell, 107:513-23, ; Little et al., 2002, Am J Hum Genet, 70:11-9). For that reason, we investigated the expression of whole Wnt family and its pathway components. Except for the regulated Wnt family members mentioned above, the expression of Wnt 5b and Wnt 10b were also expressed, but not regulated more than 2-fold. Other genes from Wnt 16-member family were not expressed.

Frizzled are a family of seven transmembrane - G protein coupled receptors to which Wnt proteins bind (reviewed in Wodarz and Nusse, 1998, Annu Rev Cell Dev Biol, 14:59-88). From 8 members of the family, higher expression was detected for Frizzled 1, 2, and 8 (Table 4). Secreted Frizzled-related sequence proteins (sFRPs) can act as decoy receptors for Wnt, and thus inhibit Wnt signaling. Very high expression of sFRP2 was detected, but not of sFRP1 and 4. sFRP2 was down-regulated at day 3, suggesting stimulation of Wnt signaling. The expression of LRP5 and its homologues was also analyzed. Low density lipoprotein receptor (LDLR) and LRP1 are strongly expressed and LRP1 was 3-fold down-regulated at day 3. LRP5 and LRP6 were also expressed and LRP5 was down-regulated to a background level at days 1 and 3. However, LRP5 mRNA was still detectable by rqRT-PCR (see below). The significance of this down-regulation is not clear at present. Possibly, its high expression is necessary very early in osteoblastic differentiation. Dickkopf is a family of proteins that extracellularly bind and regulate Wnt signaling via LRPs (Bafico et al., 2001, Nat Cell Biol, 3:683-686). Transfection experiments suggested that a Dickkopf family member constitutively inhibits LRP5 signaling, which is prevented by LRP5 mutation found in high bone mass gene (Boyden et al., 2002, N Engl J Med, 346:1513-21). However, identity of endogenous osteoblast Dickkopf is not known. We found that Dickkopf-3 was the only family member expressed at high levels in MC3T3-1 b cells (Table 4). The other components of the Wnt signaling pathway, such as β-catenin, axin, APC, LEF1 and TCF4 were also expressed. The transcription factors mediating Wnt signaling, LEF1 and TCF4, were both up-regulated. Finally, the Wnt pathway target gene TCF7 was strongly up-regulated (Table 4).

We concluded that a mixture of GP, AA and BMP-2 induces expression of several Wnt family members. The Wnt receptors and the signaling machinery are present and up-regulation of Wnt target genes indicates that the expressed Wnt are active. This is a novel aspect of osteoblast differentiation process.

### Example 6: Activation of Notch signaling pathway

The transcription factor Hey1 was strongly up-regulated: 8.5-fold at day1, and 3.3-fold at day 3 (Table 5). Hey1 is a member of a recently described family of basic helix-loop-helix (bHLH) transcription factors, related to the hairy and enhancer of split (HES). The family consists of three members: Hey1, Hey2 and HeyL (Leimeisteret et al., 1999, Mech Dev, 85:173-7). Together with HES, they are thought to be direct targets of the Notch signaling pathway (Iso et al., 2001, Mol Cell Biol,21:6071-9; Iso et al, 2002, J Biol Chem, 277:6598-607). Notch signaling is an evolutionarily conserved mechanism used by metazoans to control cell fates through local cell interactions (Artavanis-Tsakonas et al., 1999, Science, 284:770-6). Strong up-regulation of Hey1 transcription factor suggested that Notch pathway is activated in MC3T3-1 b cells during osteogenic differentiation. Therefore, we examined the expression of Notch ligands, Notch itself as a receptor, as well as factors influencing Notch activity and the target genes (Table 5). The Notch ligands Delta and Jagged, for which the probes were present on the microarray, were not detected. Nevertheless, other ligands are described for which the probes were not present. Furthermore, Notch pathway can be activated also without these ligands, through a proteolytic degradation pathway (Mumm and Kopan, 2000, Dev Biol, 228:151-165). Other factors involved in Notch processing, trafficking and regulation were expressed, such as protease ADAM10, presenilin1 and 2, and Lunatic and Radical fringe homologues (Greenwald, 1998, Genes Dev, 12:1751-62). ADAM10 expression was up-regulated 2-fold at day 3, indicating a possible activation of the Notch pathway. Notch receptor and ADAM10 are both expressed in bone (Dallas et al., 1999, Bone, 25:9-15). We concluded that activation of the Notch pathway is an exciting novel aspect of osteoblast differentiation process.

### Example 6a - BMP-2 induction of Hey1 gene:

Induction of Hey1 by BMP-2 in MC3T3 cells. MC3T3 cells are cultured in the absence (medium) or presence of GP, AA, and/or BMP-2 for 1, 2, 3 and 4 days. Total RNA is extracted and analyzed for expression of Hey1 and the control 18S ribosomal RNA by rqRT-PCR. The radioactive PCR products are analyzed by polyacrylamide gel electrophoresis and imaging by Phosphorlmager. Gel images obtained by Phoisphorlmager are shown. B: Induction of Hey1 by BMP-2 in human mesenchimal steam cells (MSC) and C2C12 cell line. Data obtained by microarray analysis. Human mesenchymal stem cells (MSC): Affymetrix array HG-U133A, probe set number 218839_at, sequence accession number N_012258; C2C12: Affymetrix array MG-U74Av2, probe set number 95671_at, sequence accession number AJ243895.

Result: Hey1 is induced by BMP-2 in murine MC3T3-pre-osteoblastic cells, as well as in dually-potent murine C2C12 cells, and human mesenchymal stem cells.

### Example 6b - siRNA mediated inhibition of Hey1 induction:

MC3T3 cells are plated on 6-well plates (0.5x10⁵ cells/well in 2 ml of medium). After 24h, siRNA is performed in a total volume of 1 ml using Oligofectamine (Life Technologies), according to the manufacturer's instructions. siRNA concentration is 0.1 µM, and the Oligofectamine amount 4 µl/well. Transfection is stopped after 4 h by adding 0.5 ml of medium containing 30% of serum, and the osteogenic stimulus. RNA is isolated after 1, 2, 3 or 4 days. For Alizarin Red S staining, medium containing 10% FCS and the osteogenic stimulus is changed twice weekly. Staining is performed after 17 days. siRNA sequences: Hey siRNA, sense strand GCTAGAAAAAGCTGAGATC, dTdT overhangs IE-HPLC purified (Xeragon Inc.). Control siRNA, sense strand AGAAGGAGCGGAATCCTCG, dTdT overhang.

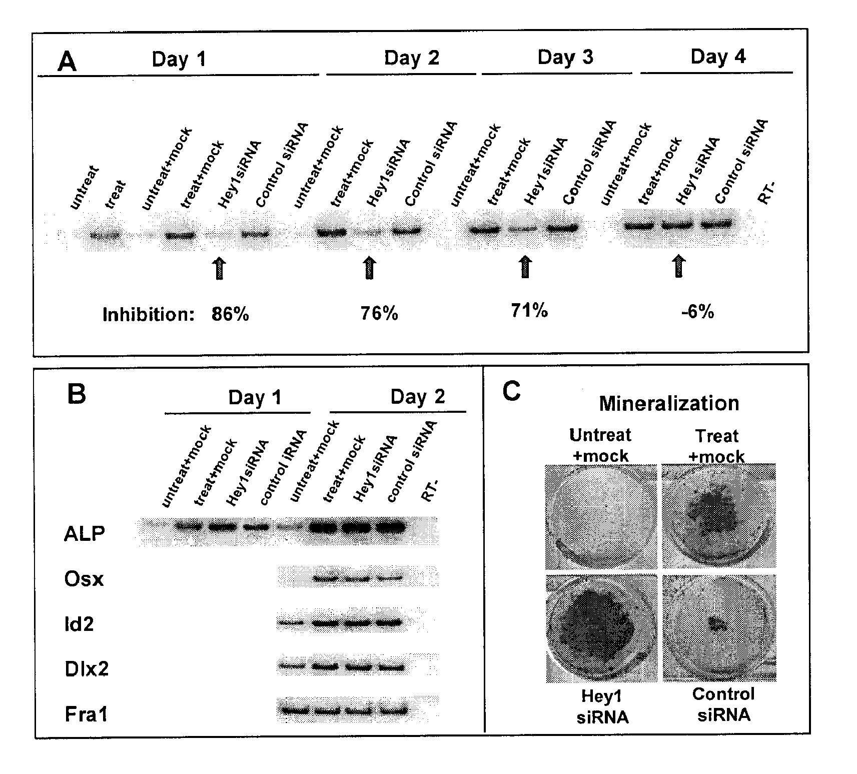
A
Day 1
Day 2
Day 3
Day 4
untreat
treat
untreat+mock
treat+mock
Hey1siRNA
Control siRNA
untreat+mock
treat+mock
Hey1siRNA
Control siRNA
untreat+mock
treat+mock
Hey1siRNA
Control siRNA
untreat+mock
treat+mock
Hey1siRNA
Control siRNA
RT-
Inhibition: 86%
76%
71%
-6%

B
Day 1
Day 2
untreat+mock
treat+mock
Hey1siRNA
control iRNA
untreat+mock
treat+mock
Hey1siRNA
control siRNA
RT-
ALP
Osx
Id2
Dlx2
Fra1

C
Mineralization
Untreat
+mock
Treat
+mock
Hey1
siRNA
Control
siRNA

Transient inhibition of BMP-2 induced Hey1 expression by Hey1 specific siRNA. MC3T3 cells are either not transfected (untreat, treat), mock transfected (untreat+mock, treat+mock) or transfected with Hey1 specific siRNA (He1 siRNA) or ineffective, control siRNA (control siRNA). After 4 h of transfection, cells are either left untreated (untreat, untreat+mock) or are treated with GP/AA/BMP-2 (treat, treat+mock, Hey1siRNA, control siRNA). Total RNA is isolated after 1, 2, 3 or 4 days and gene expression is analyzed by rqRT-PCR. B: No effect of Hey1 inhibition on the expression of early BMP-2-induced osteoblast genes by rqRT-PCR analysis. C: Transient inhibition of Hey1 induction affects mineralization process. MC3T3 cells are either mock transfected (untreat+mock, treat+mock), transfected with Hey1 specific siRNS (Hey1siRNA) or with ineffective, control siRNA (control siRNA). After 4 h of transfection, cells are either left untreated (untreat) or treated with GP/AA/BMP-2 (treat+mock, Hey1siRNA, control siRNA). Alizarin Red S staining is done after 17 days. Result: Hey1 expression can be inhibited by Hey1 siRNA, leading to little change in early gene expression, but affecting the long-term mineralization process.

### Example 6c - Cbfa1 transcriptional activity is inhibited by Hey1:

Cbfa1 and Hey1 cDNA are cloned into expression vector pcDNA3.1 (+) and used in a luciferase reporter assay. As a reporter vector OG2luc plasmid was used, consisting of minimal osteocalcin promoter and eight OSE2 Cbfa1 binding sites in front of the luciferase gene. Cbfa1 binding sites were wild type (8XOSE2 wt), or mutated to abolish Cbfa1 binding (8XOSE2 mut). Verification of Cbfa1 and Hey1 overexpression by rqRT-PCR.

Result: Cbfa1 induced 8XOSE2-dependent luciferase activity, and this effect is inhibited by coexpression of Hey1.

### Example 6d - Assay with Hey1 promoter reporter gene (to obtain antagonists or agonists to Hey1)

### a) Generation of osteoclastic cell line stably expressing Hey1 promoter luciferase

Hey1 promoter is cloned into a luciferase reporter gene vector (Rapid ResponseTM reporter vectors from Promega, Madison, WI, USA, or similar). A variety of pre-osteoblastic or pluripotent mouse cell lines is used as hosts for generation of stable cell lines: MC3T3, C2C12, CH310T1/2. Using standard techniques with antibiotics, a stable clone is selected. Further selection are based on best fold and potency of luciferase induction by BMP-2 (800 ng/ml).

### b) Cell culture for high throughput screening

All cell lines are incubated at 37°C in humidified atmosphere under 5% CO₂. C2C12 cells are routinely cultivated in DMEM HG medium supplemented with 10% FBS, 2 mM glutamine, 50 µg/ml gentamicin, 10 mM Hepes and 0.5 mg/ml G418. MC3T3 and CH310T1/2 cells are maintained in α-MEM supplemented with 10% FCS, 2 mM L-glutamine, 100 IU/ml penicillin, 100 µg/ml streptomycin and 0.75 mg/ml G418. For the reporter gene experiments, all the clones are grown in the absence of G418.

BMP-2 is stored as 500 µg/ml stock solution in water at -80°C and after thawing at 4°C. Final concentration is 800 ng/ml. For high controls with BMP-2, 500 µg/ml BMP-2 is diluted 1:250 in culture medium containing 1.25 % DMSO and 20 µl were added to 30 µl of cell suspension in wells on 384-well plate. Final concentration was 800 ng/ml of BMP-2. Low controls are done with medium containing 1.25 % DMSO (BMP-2 controls) or with medium containing 0.63 % DMSO (TGF-b1 controls). Twenty µl is added to 30 µl of cell suspension in wells on a 384-well plate.

### c) Reporter gene assay for high throughput screening

The cells in triple flasks are washed with 30 ml phosphate-buffered saline (PBS). Trypsin/EDTA solution is added (15 ml) and incubated with cells for 1-2 min at 37°C. The culture medium is added (15 ml) and the cells are collected by centrifugation. The cells are counted and diluted to 6.7x10³ cells/ml (C2C12) or 1.78x10⁴ cells/ml (MC3T3 and CH310T1/2). Fifty µl of this suspension is added to wells in Greiner 384-well plates with the Multidrop machine in sterile conditions. The cell suspension is constantly mixed during distribution with the Multidrop. The Multidrop cassette is maintained sterile with 70 % ethanol. The final cell number per well is 333 (C2C12-15a) and 888 (MC3T3 and CH310T1/2). The cells are grown for 3 days until confluency at 37°C in a tissue culture incubator. The compounds or reference stimulators (BMP-2) are added to the confluent cells using Multidrop and Cybiwell. The incubation is continued for another 24 h.

Luciferase activity is measured as follows: plates are brought from 37°C to the room temperature and 25 µl of SteadyGlo reagent is added to each well. The plates are incubated for 60 min at room temperature and luminescence is measured at Viewlux luminometer (60 s measurement time, binning factor 2, medium speed, medium gain).

### d) Criteria for hit identification in high throughput screen

Compounds inducing significant activation or reduction (agonists and antagonists) of luciferase (usually >2-fold induction) are taken as a first set of hits. The compounds are confirmed in dose response curves with at least 9 concentration points. Compounds producing regular sigmoidal curves are taken as first priority hits.

Note: Agonists and Antagonists of Hey-1 are interesting for bone loss diseases as antagonists stimulate osteoblast differentiation (direct implication); and Agonists keep mesenchymal stem cells in their pluripotent stage, thus generating higher numbers of cells that can be stimulated to differentiate with endogenous factors (indirect implication).

### Example 7: Other interesting regulated genes

Several additional up-regulated genes not belonging to the above groups will be mentioned: FKBP5, RAMP1, and three enzymes from the cholesterol biosynthesis pathway. The criteria for their selection were: very strong up-regulation, up-regulation of several genes from the same pathway, and a relationship with very recent findings in bone biology. These genes are already included in the Table 1, either as a part of big superfamilies, or in the group of Other genes.

FKBP5 belongs to the family of immunophilins, a group of intracellular receptors for immunosuppresive drug FK506. Upon binding of FK506, FKBPs inhibit Ca²⁺-dependent protease calcineurin. FKBPs have intrinsic peptidyl/prolyl cis/trans isomerase activity (reviewed in Gothel and Marahiel, 1999, Cell Mol Life Sci, 55:423-36). Recent report showed that FK506 enhances osteoblast differentiation process in mesenchymal cells (Tang et al., 2002, Cell Biol Int, 26:75-84), suggesting a connection between FKBPs and osteoblasts. We observed 7-fold increase in the expression of FKBP5 at day 3 (Table 1, Others).

Together with the calcitonin receptor-like receptor, the receptor activity-modifying protein 1 (RAMP1) forms the functional receptor for calcitonin gene-related peptide, (CRLR) (McLatchie et al., 1998, Nature 393:333-9). Striking up-regulation of RAMP1 by 30-fold was detected at day 3 (Table 1, Receptors). The change in RAMP1 expression during osteoblast differentiation is a novel finding.

Three genes coding for enzymes involved in cholesterol biosynthesis were up-regulated. Rate-limiting enzyme HMG CoA reductase is up-regulated 11-fold, suggesting importance of cholesterol or some of its precursors in the osteoblast differentiation process (Table 1, Others). Sterol-C5-desaturase and dehydrocholesterol reductase were up-regulated 3-fold. The involvement of mevalonate pathway of cholesterol production and geranylgeranyl modification of proteins has been identified as a target of bisphosphonates in the bone resorbing cells osteoclasts (Coxon et al., 2000, J Bone Miner Res, 15:1467-1476). In osteoblastic cells, the mevalonate pathway was implicated in the mode of action of statins, which were in some systems shown to have a bone anabolic effects (Garrett et al., 2001, Curr Pharm Des, 7:65-70). Furthermore, the identification of the mutated LRP5 as high bone mass gene (Little et al., 2002, Am J Hum Genet, 70:11-9) raised the interest in other related low density lipoprotein receptor genes and their ligands.

### Example 8: Confirmation of the gene expression profiles by rqRT-PCR

We have independently confirmed observed regulation of 16 genes by rqRT-PCR, 10 of them coding for transcription factors. From most groups discussed above, we selected few representatives: for BMP-2 signaling pathway -SMAD1 and 6, for BMP-regulated genes - JunB, Id2 and DIx2, for osteoblast differentiation related genes - Fra1, for TGFβ signaling pathway - TGFβ1, TGFβ3 and TIEG, for Wnt signaling pathway - Wnt6, TCF7 and LRP5, for Notch signaling pathway - Hey1, for other interesting genes - FKBP5, RAMP1 and Hlx (see Table 1). For all the genes analyzed, the expression profiles obtained by rqRT-PCR closely corresponded to the profiles obtained by microarray analysis. Together with osteoblast markers and a housekeeping gene, the number of analyzed and confirmed genes was 24, indicating high reliability of gene regulation by microarrays.

**(a) Table 1. Functional classification of genes regulated during osteoblastic differentiation of MC3T3-1b cells.**

| **Matrix Proteins and Adhesion** | | | | |
|---|---|---|---|---|
| **Name** | **Description** | **Accession number** | **Relative expression level** | |
| | | | **Day 1** | **Day 3** |
| **Bone sialoprotein** | Integrin binding sialoprotein | L20232 | 1.00 | 12.85 |
| **Osteocalcin** | Bone gamma carboxyglutamate protein 1 | L24431 | 1.00 | 10.20 |
| **Osteomodulin** | Proteoglican, bone matrix protein | AB007848 | 0.97 | 4.42 |
| **TGFBI. Kerato-epithelin** | Extracellular adhesion molecule,binds to collagen 1, 2 and 4. TGF-beta induced | L19932 | 3.49 | 2.05 |
| **Sprr2** | Cornified cell envelope of stratified squamosus epithelia | AJ005569 | 1.80 | 3.25 |
| **Tenascin C** | Extracellular matrix glycoprotein. Secreted by OB. Stimulate OB differentiation. TGF-beta induced | X56304 | 2.53 | 2.83 |
| **Vinculin** | Cytoplasmic face of adhesion plaques | L18880 | 0.83 | 2.76 |
| **Periplakin** | Component of the cornified envelope of keratinocytes | AF013715 | 0.71 | 2.36 |
| **Peptidase 4 / prolidase** | Recycling of proline for collagen synthesis and cell growth | U51014 | 1.28 | 2.25 |
| **PG-M** | Chondroitin sulfate proteoglycan 2 | D45889 | 2.12 | 0.65 |
| **Pinin** | Desmosome-associated protein | Y08701 | 1.43 | 2.10 |
| **SPRR2A. Small proline-rich protein 2A** | Cross-linked envelope protein of **keratinocytes** | AJ005559 | 0.23 | 2.00 |
| **Cadherin 11 (OB- cadherin)** | Calcium-dependent adhesion protein | D21253 | 0.84 | 0.49 |
| **NID. Nidogen 1 (entactin)** | Basement membrane protein | L17324 | 0.99 | 0.48 |
| **VCAM-1** | Vascular cell adhesion molecule 1 | M84487 | 0.64 | 0.45 |
| **CHST2** | Carbohydrate (chondroitin 6/keratan) sulfotransferase 2 | AB011451 | 1.20 | 0.44 |
| **Lysyl oxidase-2** | Extracellular copper-dependent enzyme. Initiates cross-linking of collagens and elastin | U79144 | 1.02 | 0.44 |
| **JUP. Junction plakoglobin** | Common junctional plaque protein | M90365 | 0.44 | 0.54 |
| **Osteoglycin** | Small leucine-rich proteoglycan. In conjunction with TGFbeta1 orTGFbeta2, induces bone formation | D31951 | 0.43 | 0.45 |
| **Glypican 4** | Cell surface heparan sulfate proteoglycan | X83577 | 0.66 | 0.42 |
| **Matrilin 4** | Extracellular matrix protein | AJ010984 | 0.41 | 0.43 |
| **Laminin B1** | Major component of basal laminae | X05212 | 0.35 | 0.40 |
| **Thrombospondin 2** | Extracellular matrix glycoprotein | L07803 | 0.57 | 0.36 |
| **ALCAM** | Activated leukocyte cell adhesion molecule | L25274 | 0.36 | 0.95 |
| **Pactolus** | Integrin beta subunit-like cell surface protein | AF051367 | 0.34 | 1.00 |
| **Fibulin 2** | Extracellular matrix protein, binds to fibronectin | X75285 | 0.54 | 0.33 |
| **P4HA1** | Post-translational formation of 4-hydroxyproline in collagens | U16162 | 0.33 | 0.47 |
| **NID2 Nidogen 2 (osteonidogen, entactin-2)** | Basement membrane protein | AB017202 | 0.33 | 0.33 |
| **VCAM-1** | Vascular cell adhesion molecule-1 | U12884 | 0.50 | 0.31 |
| **T-VCAM-1** | Vascular cell adhesion molecule-1 truncated form | U12884 | 0.53 | 0.27 |
| **Latent TGF beta binding protein 2.** | Structural extracellular matrix protein for targeting TGF-beta action. | AF004874 | 1.21 | 0.26 |
| **Fibrillin 1** | Major constituent of the extracellular microfibrils | L29454 | 0.62 | 0.26 |
| **Procollagen, type IV, alpha 2** | Basement membrane constituent | X04647 | 0.64 | 0.26 |
| **MMP13** | Collagenase-3 | X66473 | 0.87 | 0.22 |
| **Procollagen, type IV, alpha 1** | Basement membrane constituent | M15832 | 0.35 | 0.20 |
| **AOC3. Amine oxidase, copper containing 3** | Vascular adhesion protein 1 | AF078705 | 0.37 | 0.14 |
| **ENG. Endoglin** | Major glycoprotein of vascular endothelium. Binds TGF-beta | X77952 | 0.14 | 0.68 |
| | | | | |

| **Growth Factors** | | | | |
|---|---|---|---|---|
| **Name** | **Description** | **Accession number** | **Relative expression level** | |
| | | | **Day 1** | **Day 3** |
| **Wnt10a** | Signaling molecule, secreted and associated with extracellular matrix | U61969 | 1.60 | 6.80 |
| **PPY. pancreatic polypeptide** | Pancreatic hormone. synthesized in pancreatic islets of Langerhans. Regulator of pancreatic and gastrointestinal functions. | M18208 | 1.00 | 4.80 |
| **TGFbeta1. Transforming growth factor beta 1** | Regulator of OB proliferation and differentiation | AJ009862 | 4.56 | 3.56 |
| **Wnt6** | Signaling molecule, secreted and associated with extracellular matrix | M89800 | 1.74 | 3.85 |
| **PDGF alpha. Platelet derived growth factor, alpha** | Regulator of OB proliferation and differentiation | M29464 | 1.04 | 2.70 |
| **IGFBP10. Insulin-like growth factor binding protein 10** | Modulate the growth promoting effects of the IGFs | M32490 | 2.70 | 1.61 |
| **Wnt1** | Signaling molecule, secreted and associated with extracellular matrix | M11943 | 2.30 | 1.00 |
| **SEMA3A. Semaphorin IIIa, collapsin I** | Needed for normal patterning and growth of nerves, bones and heart | D85028 | 0.79 | 2.18 |
| **TGFbeta3** | Regulator of OB proliferation and differentiation | M32745 | 2.10 | 1.12 |
| **Cardiotrophin-1** | IL-6 superfamily. Binds to and activates the LIFR/gp 130 receptor complex | U18366 | 0.49 | 1.15 |
| **Mac2 (galectin-3)** | Galactose- and IgE-binding lectin secreted by inflammatory macrophages and in colonic epithelium | X16834 | 0.46 | 0.94 |
| **IGFBP2. Insulin-like growth factor binding protein 2** | Inhibit or stimulate the growth promoting effects of the IGFs | X81580 | 0.46 | 0.81 |
| **SCYD1 (fractalkine, neurotactin)** | Small inducible cytokine subfamily D member 1. Regulating leukocyte adhesion and migration processes at the endothelium | U92565 | 0.63 | 0.45 |
| **Small inducible** cytokine **A7** | Chemotactic factor for monocytes | X70058 | 0.74 | 0.45 |
| **IGFBP6. Insulin-like growth factor binding protein-6** | Inhibits or stimulates the growth promoting effects of the IGFs | X81584 | 0.81 | 0.45 |
| **BMP-4** | TGF-beta superfamily. Stimulates OB differentiation | L47480 | 0.44 | 0.77 |
| **TGFbeta2** | Regulator of OB proliferation and differentiation | X57413 | 0.95 | 0.43 |
| **M-CSF** | Macrophage colony-stimulating factor. Stimulates osteoclastogenesis. | M21952 | 0.74 | 0.41 |
| **VEGF- D. Vascular endothelial growth factor D** | Stimulates endothelial cell proliferation. Stimulates angiogenesis | X99572 | 0.99 | 0.36 |
| **Phosphohexose isomerase (PHI)** | Ectoenzyme/exoenzyme family; glycolysis and gluconeogenesis pathways; Upon secretion-cytokine with tumor autocrine motility factor (AMF), neuroleukin (NLK) and maturation factor (MF) functions | M14220 | 0.36 | 0.64 |
| **Fibroblast growth factor 7** | Keratinocyte growth factor | Z22703 | 0.38 | 0.36 |
| **Angiotensinogen** | Inhibits OB differentiation (*in vitro*) | AF045887 | 0.56 | 0.26 |
| **Fibroblast growth factor 18** | Stimulates hepatic and intestinal cell proliferation | AB004639 | 0.45 | 0.23 |
| **Parathyroid hormone-like peptide (PTHRP)** | Supports osteoclastogenesis, osteolytic bone metastases and cartilage-matrix degradation during endochondral bone formation | M60057 | 0.23 | 0.18 |
| **Gremlin** | Secreted. BMP antagonist | AF045801 | 0.12 | 0.15 |
| | | | | |

| **Receptors** | | | | |
|---|---|---|---|---|
| **Name** | **Description** | **Accession number** | **Relative expression level** | |
| | | | **Day 1** | **Day 3** |
| **PTHR-Parathyroid hormone receptor** | GPCR | X78936 | 2.55 | 33.85 |
| **RAMP1** | Transports calcitonin-receptor-like receptor to the plasma membrane where it acts as a calcitonin-gene-related peptide receptor | AJ250489 | 1.00 | 30.00 |
| **LIFR-Leukemia inhibitory factor receptor** | Cytokine family of receptors. | D17444 | 2.21 | 5.92 |
| **LEPR. Leptin receptor** | Cytokine family of receptors. | U42467 | 1.00 | 4.95 |
| **EPHA2. Ephrin type A receptor 2** | Receptor tyrosine kinase | U07634 | 4.85 | 0.96 |
| **EPHB2. Ephrin type B receptor 2** | Receptor tyrosine kinase | L25890 | 3.95 | 2.30 |
| **OPG. Osteoprotegerin** | Tumor necrosis factor receptor superfamily, member 11b. Inhibits OC genesis | U94331 | 1.27 | 3.84 |
| **SSTR4. Somatostatin receptor type 4** | GPCR | U26176 | 0.42 | 3.26 |
| **PTGFR. Prostaglandin** F **receptor** | GPCR | D17433 | 1.33 | 3.14 |
| **FGFR2. Fibroblast growth factor receptor 2** | Receptor tyrosine kinase | M63503 | 1.57 | 2.91 |
| **SSTR2. somatostatin receptor type 2** | GPCR. TGF beta inducible | AF008914 | 2.45 | 2.85 |
| **THRA. Thyroid hormone receptor alpha** | Nuclear receptor | U09504 | 2.15 | 2.60 |
| **ROR alpha. RAR-related orphan receptor alpha** | Orphan nuclear receptor. | U53228 | 1.55 | 2.60 |
| **PLAUR-plasminogen activator, urokinase receptor** | Localizing and promoting plasmin formation | X62700 | 2.06 | 2.50 |
| **NRP1. Neuropilin 1, VEGF165 receptor** | Cell surface glycoprotein. Receptor for Semaphorin III | D50086 | 2.13 | 1.27 |
| **MERTK. C-mer proto-oncogene tyrosine kinase** | Receptor tyrosine kinase | U21301 | 0.48 | 1.50 |
| **GHR. Growth hormone receptor** | Cytokine family of receptors | M31680 | 0.46 | 0.98 |
| **IL4R. Interleukin-4 receptor (secreted form)** | Cytokine family of receptors | M27960 | 0.49 | 0.44 |
| **VEGFR2. Vascular endothelial growth factor receptor 2** | Receptor tyrosine kinase | D88689 | 0.89 | 0.43 |
| **Notch homologue 1** | | Z11886 | 0.54 | 0.43 |
| **NTRK3. Neurotrophin-3 receptor** | Receptor tyrosine phosphatase | AF035400 | 0.76 | 0.43 |
| **LRP1. Low density lipoprotein receptor related protein 1** | Alpha-2-macroglobulin receptor | X67469 | 0.83 | 0.38 |
| **OSMR. Oncostatin M receptor beta subunit** | Cytokine family of receptors | AB015978 | 0.78 | 0.37 |
| **sFRP-2. Secreted frizzled related protein 2** | Modulator of Wnt signaling | U88567 | 0.94 | 0.34 |
| **PDGFR alpha. Platelet derived growth factor receptor, alpha** | Receptor tyrosine kinase | M57683 | 0.48 | 0.33 |
| **Notch gene homologue 3** | | X74760 | 0.37 | 0.27 |
| **LRP5. Low density lipoprotein receptor**-**related protein 5** | Component of the Wnt receptor complex. | AF064984 | 0.25 | 0.32 |
| **Or6 gene.** | Olfactory receptor of the OR37 subfamily. GPCR | AJ133430 | 0.20 | 1.00 |
| **MPT** | Receptor tyrosine phosphatase | D13903 | 0.50 | 0.20 |
| **RDC1. Chemokine orphan receptor 1** | GPCR | AF000236 | 0.41 | 0.13 |
| **PTGER1. Prostaglandin E receptor 1 (subtype EP1)** | GPCR | Y07611 | 1.60 | 0.11 I |
| | | | | |

| **Cytoskeleton** | | | | |
|---|---|---|---|---|
| **Name** | **Description** | **Accession number** | **Relative expression level** | |
| | | | **Day 1** | **Day 3** |
| **KIFC1** | Kinesin family motor protein C1 | D49544 | 1.62 | 3.08 |
| **Alpha-tubulin** | Testis-specific | M13443 | 1.63 | 2.91 |
| **KIF4** | Kinesin family motor protein 4 | D12646 | 1.49 | 2.82 |
| **Vinculin** | Cytoplasmic face of adhesion plaques | L18880 | 0.83 | 2.76 |
| **Dia2 Diaphanous-related formin** | RhoA binding protein | AF094519 | 1.40 | 2.50 |
| **NDPP-1** | Microfilament assembly and cell motility | D10727 | 2.36 | 0.82 |
| **LIMK** | LIM-domain containing, Ser/Thr protein kinase. Activated by Rho small GTPase. Regulates actin cytoskeletal reorganization | U15159 | 2.35 | 2.20 |
| **Eg5** | Kinesin-related mitotic motor protein | AJ223293 | 1.28 | 2.11 |
| **Myosin** X | | AJ249706 | 1.23 | 2.02 |
| **ARHGAP5** | Rho GTPase activating protein 5 | U67160 | 0.48 | 1.00 |
| **LSP1. Lymphocyte-specific protein 1** | Cytoskeleton-associated protein | D49691 | 0.47 | 0.80 |
| **VIL2. Vilin** | Connections of major cytoskeletal structures to the plasma membrane | X60671 | 0.39 | 0.55 |
| | | | | |

| **Signaling** | | | | |
|---|---|---|---|---|
| **Name** | **Description** | **Accession number** | **Relative expression level** | |
| | | | **day 1 +/-** | **day 3 +/-** |
| **CAPN6. Calpain 6.** | Ca2+-dependent intracellular non-lysosomal protease believed to participate in signal transduction | Y12582 | 1.00 | 4.05 |
| **IFI204. Interferon activated gene 204** | Probably mediates growth inhibitory effect of interferon | M31419 | 2.50 | 1.00 |
| **ADAM10. A disintegrin and metalloprotease domain 10** | Cell-cell and cell-matrix interaction. Controls proteolytic processing of Notch | AF011379 | 0.87 | 2.32 |
| **GRB. Growth factor receptor bound protein 10** | Plays a functional role in insulin and IGF-I signaling | AF022072 | 0.64 | 2.08 |
| **SOCS1** | Cytokine inducible negative regulator of Janus kinase/STAT signaling | U88325 | 2.04 | 1.21 |
| **pip92** | Immediate early gene. Activated by different MAPK pathways by growth factors or environmental stress. | M59821 | 0.94 | 2.03 |
| **CAPNS. Calpain 5** | Ca2+-dependent intracellular non-lysosomal protease believed to participate in signal transduction | Y10656 | 0.74 | 0.49 |
| **IZPR2. Inositol trisphosphate receptor type 2** | Coupled to Ca2+ channels. Release of Ca2+ from intracellular stores | AF031127 | 0.74 | 0.48 |
| **PIK3R1** | p85, regulatory subunit of PI3K | U50413 | 0.46 | 0.88 |
| **KSR1** | PK. Positive modulator of Ras-dependent signaling | U43585 | 0.45 | 1.00 |
| **CaIDAG-GEFI** | Rap guanine nucleotide exchange factor. Rap-stimulate or inhibit MAPK pathway | U78171 | 0.44 | 1.00 |
| **SOCS-2. Suppressor of cytokine signalling-2** | Jak/STAT signaling inhibitor. Suppresses GH and IGF-1 | U88327 | 0.41 | 0.40 |
| **Ndr1. N-myc downstream regulated 1** | Involved in stress responses, hormone responses, cell growth and differentiation | U52073 | 0.52 | 0.39 |
| **TPD52L1. Tumor protein D52-like 1** | Calcium-mediated signal transduction and cell proliferation | AF004428 | 0.77 | 0.35 |
| **ADCY7. Adenylate cyclase 7** | Membrane-bound, Ca+2 inhibitable adenylate cyclase | U12919 | 0.59 | 0.34 |
| **NEDD9 (cas I)** | Docking protein. Plays a central role for tyrosine-kinase-based signaling related to cell adhesion | AF009366 | 1.30 | 0.34 |
| **ACK2** | Non-receptor protein tyrosine kinase. Associated with integrin signaling. Cdc42-regulated | AF037260 | 0.59 | 0.31 |
| **GBP2** | Guanylate binding protein 2, interferon-inducible | AJ007970 | 0.31 | 0.27 |
| **GOA-alpha** | Guanine nucleotide-binding protein Go, alpha subunit | M36777 | 0.50 | 0.23 |
| **R-Ras** | Small GTPase. Affects cell adhesion by maintaining integrin activity | M21019 | 0.19 | 0.53 |
| **WISP-2** | Wnt-1-induced signaling protein. Connective tissue growth factors family | AF100778 | 0.21 | 0.16 |
| **NOV. Nephroblastoma overexpressed gene** | Insulin-like growth factor binding protein family | Y09257 | 0.19 | 0.15 |
| **CRABP2. Cellular retinoic acid binding protein II.** | Regulates the access of retinoic acid to the nuclear retinoic acid receptors. | M35523 | 0.14 | 0.34 |
| **PLCbeta1** | Phospholipase C beta1. Activated by G alpha-q and G alpha-11 | U85714 | 0.13 | 4.21 |
| **TRAF5** | Tumor necrosis factor (TNF) receptor-associated factor 5 | D78141 | 1.00 | 0.11 |
| | | | | |

| **Kinases** | | | | |
|---|---|---|---|---|
| **Name** | **Description** | **Accession number** | **Relative expression level** | |
| | | | **Day 1** | **Day 3** |
| **Serine/threonine kinase** 5 | Possible role in cell growth | D21099 | 0.08 | 5.65 |
| **esk/TTK kinase** | Ser/Thr/Tyr (dual-specificity) kinase, in rapidly proliferating cell lines | M86377 | 1.53 | 3.16 |
| **LYN** | Tyr protein kinase, belongs to Src family | M57696 | 1.69 | 2.91 |
| **EEF2K** | Eukaryotic elongation factor-2 kinase | U93848 | 1.00 | 2.85 |
| **BUB1** | Mitotic checkpoint Ser/Thr-protein kinase | AF002823 | 1.45 | 2.84 |
| **Mst1. Ste20-like protein kinase** | Stress-responsive protein kinase | U28726 | 1.15 | 2.77 |
| **FRK (fyn-related kinase)** | Tyr-protein kinase, related to Src family. | Z48757 | 2.70 | 0.54 |
| **STK6** | Ser/Thr kinase 6 -mitotic centosomal protein kinase | U80932 | 1.75 | 2.62 |
| **PLK Polo-like kinase homologue** | Ser/Thr kinase, required for mitosis | U01063 | 1.94 | 2.37 |
| **LIMK** | Ser/Thr protein kinase. Regulates actin cytoskeletal reorganization | U15159 | 2.35 | 2.20 |
| **Calcium/calmodulin-dependent protein kinase II delta** | Ser/Thr protein kinase | AF059029 | 0.40 | 2.35 |
| **NEK2** | Ser/Thr kinase. Mitotic regulation | AF013166 | 1.43 | 2.29 |
| **RIPK1** | Ser/Thr protein kinase. Required for TNFR1 activation of NF-kappa b | U25995 | 0.67 | 2.10 |
| **STK18** | Ser/Thr kinase 18. Polo-family of mitotic regulators | L29480 | 1.47 | 2.01 |
| **MEK1** | Dual specificity mitogen-activated protein kinasekinase 1 | L02526 | 1.07 | 0.49 |
| **DMPK. Dystrophia myotonica kinase** | Important role in Ca2+ homeostasis and signal transduction system. Mutations in myotonic dystrophy | Z38015 | 0.35 | 0.99 |
| | | | | |

| **Transcription Factors** | | | | |
|---|---|---|---|---|
| **Name** | **Description** | **Accession number** | **Relative expression level** | |
| | | | **Day 1** | **Day 3** |
| **TCF7** | Target of Wnt signaling. | X61385 | 0.93 | 8.25 |
| **Hey1** | bHLH transcription factor. Notch target gene | AJ243895 | 8.05 | 3.30 |
| **Mybl2** | Cell cycle progression | X70472 | 7.90 | 5.30 |
| **LEF1. Lymphoid enhancer binding factor 1** | Activation of Wnt responsive genes | D16503 | 1.00 | 7.85 |
| **junB** | BMP target gene | U20735 | 5.00 | 6.05 |
| **LEF1. Lymphoid enhancer binding factor 1** | Activation of Wnt responsive genes | D16503 | 1.00 | 4.85 |
| **Oct-11** | POU domain, class 2 | Z18537 | 3.25 | 4.30 |
| **Msx2** | Homeobox domain, msh-like 2. Increased in early OB differentiation | X59252 | 4.20 | 1.30 |
| **Id1. Inhibitor** of DNA **binding 1** | HLH transcriptional inhibitor. BMP-2 inducible | M31885 | 4.00 | 0.67 |
| **Id2. Inhibitor of DNA binding 2** | HLH transcriptional inhibitor. BMP-2 inducible | AF077861 | 3.97 | 2.01 |
| **EGR1/Krox 24. Early growth response 1** | Up-regulated in OB after mechanical loading; Egr1-/- mice: induced production of M-CSF, stimulation of osteoclasts | M28845 | 0.33 | 3.62 |
| **HOXC9** | Homeobox transcription factor | X55318 | 3.55 | 1.54 |
| **Fosl1(Fra-1)** Fos-like **antigen** 1 | AP-1 component. Involved in OB and osteoclast differentiation | AF017128 | 3.40 | 1.00 |
| **Id3. Inhibitor of DNA binding 3** | HLH transcriptional inhibitor. BMP-2 inducible | M60523 | 3.01 | 3.25 |
| **Dlx2** | Homeobox. Craniofacial patterning and morphogenesis, development of ventral forebrain | M80540 | 3.16 | 1.39 |
| **HFH-11** | Fork head/ winged-helix transcription factor | Y11245 | 1.63 | 2.89 |
| **CLOCK** | bHLH transcription factor | AF000998 | 2.71 | 0.87 |
| **SMAD6** | TGF-beta/BMP signaling pathway, inhibitory molecule | AF010133 | 2.63 | 1.36 |
| **Zac1** | Zn finger protein. Promotes cell cycle arrest and apoptosis; act as a positive or negative transcriptional cofactor for nuclear receptors | X95503 | 1.45 | 2.57 |
| **Hlx** | Homeobox transcription factor. Involved in embryogenesis and hematopoiesis | X58250 | 2.56 | 1.09 |
| **BRCA1** | Zn finger superclass. Transcriptional regulation of p21 in response to DNA damage | U32446 | 2.25 | 2.42 |
| **EGR2/Krox20.** Early **growth response 2** | Zn finger superclass. Transition from the promyelinating to the myelinating stage of Schwann cell development | M24377 | 0.70 | 2.27 |
| **SMAD7** | TGF-beta/BMP signaling pathway, inhibitory molecule | AF015260 | 2.25 | 0.88 |
| **MafG** | Transcriptional modulator. Interaction with various basic-leucine zipper-type transcription factors. | AB009693 | 1.00 | 2.20 |
| **TIEG** | TGF-beta inducible. Transcriptional repressor | AF064088 | 2.18 | 1.91 |
| **Mrg1a** | TALE homeobox protein subfamily | U68383 | 1.00 | 2.10 |
| **c-myc** | BHLH transcription factor | L00039 | 0.96 | 2.09 |
| **SMAD1** | BMP signaling pathway, stimulatory molecule | U58992 | 1.38 | 2.08 |
| **PML** | Zn-finger nuclear protein. Tumor suppressor, regulates p53 response to oncogenic signals | U33626 | 2.07 | 1.10 |
| **TCF4. Transcription factor 4** | HMG box. Activation of Wnt responsive genes | AF107298 | 1.04 | 2.03 |
| **AT motif binding factor 1** | Multiple homeobox domain, zinc finger transcription factor | D26046 | 0.50 | 0.56 |
| **HMX3** | Homeobox transcription factor | X75330 | 1.00 | 0.48 |
| **FHL1. Four and a half LIM domains protein 1** | Involvement in muscle development or hypertrophy | U41739 | 0.51 | 0.48 |
| **Oct1** | POU2F1 (POU domain, class 2, transcription factor 1). Transcription factor for small nuclear RNA and histoneH2b genes. | X68363 | 0.51 | 0.47 |
| **FHL2. Four and a half LIM domains protein 2** | Transcriptional coactivator. Transmits Rho **signals** | AF055889 | 0.74 | 0.45 |
| **ZFP97** | Kruppel family of Zn finger proteins | L20450 | 0.43 | 1.13 |
| **HIC-1. Hypermethylated in cancer 1** | BTB/POZ transcriptional repressors | AJ007511 | 1.00 | 0.43 |
| **NFI-B2** | Member of NFI/CTF family | D90173 | 0.42 | 0.93 |
| **PBX1** | TALE homeobox protein subfamily. Pre B-cell leukemia transcription factor | L27453 | 0.84 | 0.35 |
| **Six-1.Sine oculis-related homeobox 1** | Six homeobox proteins control muscle formation through activation of one of its key regulators, myogenin | X80339 | 0.46 | 0.32 |
| **Eos** | Icarus family of transcription factors. Lymphoid development | AB017615 | 0.29 | 1.00 |
| **ISGF3G** | Interferon dependent | U51992 | 0.28 | 0.31 |
| **p51/p73L/p63/p40** gene | p53 homologue, modulates p53 function | AB010152 | 0.34 | 0.27 |
| **AP-2** | Developmental regulation | U17291 | 0.32 | 0.26 |
| **FOG. Friend of GATA-1** | Cofactor for transcription factor GATA-1 in erythroid and megakaryocytic differentiation | AF006492 | 1.53 | 0.25 |
| **Msx1** | Homeobox domain, msh-like 1. Early OB differentiation | X14759 | 1.05 | 0.25 |
| **IRF5 Interferon regulatory factor** 5 | Helix-turn-helix transcription factor, involved in innate immune response to infection | AF028725 | 1.79 | 0.21 |
| **Mel-18** | Polycomb group of proteins, regulates expression of Homeobox genes | D90085 | 0.21 | 1.00 |
| **HoxB8** | Homeobox transcription factor. Part of a developmental regulatory system | M18399 | 0.20 | 0.93 |
| **Id4** | HLH transcriptional inhibitor | AJ001972 | 0.49 | 0.13 |
| **ATFx** | Leucine-zipper protein. Interact with G-CSF gene promoter element 1-binding protein | AB012276 | 0.11 | 0.31 |

| | | | | |
|---|---|---|---|---|
| **Cell cycle and** | | | | |
| **Apoptosis** | | | | |
| **Name** | **Description** | **Accession number** | **Relative expression level** | |
| | | | **Day 1** | **Day 3** |
| **Cyclin D1** | G1/S transition control | M64403 | 5.90 | 2.49 |
| **CDC25C** | Protein tyrosine phosphatase. Progression of cell cycle | U15562 | 1.27 | 3.44 |
| **GTSE-1** | G2 and S phase expressed protein | AJ222580 | 1.85 | 3.11 |
| **Caspase 7** | Apoptosis-related cysteine protease | U67321 | 1.84 | 3.04 |
| **Sha1** | S-M checkpoint control | AF062378 | 1.06 | 2.70 |
| **CDCREL-1 homologue** | Cell division control | AF033350 | 2.70 | 1.49 |
| **Survivin -BIRC5** | Inhibitor of caspase 3 and 7 | AB013819 | 1.26 | 2.57 |
| **MyD118** | Negative growth control | X54149 | 2.53 | 1.65 |
| **CDC25C** | Dosage-dependent inducer in mitotic control | L16926 | 1.38 | 2.15 |
| **Cyclin A2** | Cell cycle control | X75483 | 1.55 | 2.11 |
| **RIPK1** | Ser/Thr protein kinase. Initiates apoptosis. | U25995 | 0.67 | 2.10 |
| **Ect2** | Regulation of cytokinesis | L11316 | 1.23 | 2.07 |
| **Siah-1** | p-53 inducible mediator of cell cycle arrest | Z19580 | 2.00 | 1.40 |
| **BNIP3L** | Nuclear gene encoding mitochondrial protein. induces apoptosis | AF067395 | 0.49 | 0.68 |
| **DID** | Induces ice-like proteases and apoptosis | U75506 | 0.33 | 0.47 |
| **GAS2** | Growth arrest specific 2 | M21828 | 0.45 | 0.46 |
| **GAS1** | Growth suppression. Blocks entry to s phase | X65128 | 0.89 | 0.38 |
| **Bcl-2** | Suppresses apoptosis | L31532 | 0.33 | 0.75 |
| **Proliferin** | Secreted protein. Stimulate cell proliferation | K03235 | 1.00 | 0.29 |
| **GAS6** | Growth-arrest | X59846 | 1.00 | 0.24 |
| | | | | |

| **DNA Replication** | | | | |
|---|---|---|---|---|
| **Name** | **Description** | **Accession number** | **Relative expression level** | |
| | | | **Day 1** | **Day 3** |
| **NASP, nuclear autoantigenic sperm protein** | Transporting newly synthesized histones to the nucleus. Testis and sperm-specific. | AF034610 | 1.71 | 5.55 |
| **BIm** | ATP dependent DNA helicase, involved in DNA replication and repair | AB008674 | 5.50 | 3.45 |
| **IMPDH1: Inosine monophosphate dehydrogenase type I** | Rate limiting enzyme in de novo synthesis of guanine nucleotide | U00978 | 1.61 | 5.40 |
| **CDC45-related protein** | Initiation of DNA replication | AF098068 | 4.40 | 1.00 |
| **HMG2 High mobility group protein 2** | Non-histone structural protein of chromatine | X67668 | 1.56 | 3.25 |
| **CDC46** | Initiation of DNA replication | D26090 | 2.67 | 3.07 |
| **CHAFA** | Chromatin assembly factor-I p150 subunit | AJ132771 | 2.35 | 2.76 |
| **ORC1-related protein** | Initiation of DNA replication | AJ003133 | 2.65 | 1.63 |
| **CDC6-related protein** | Initiation of DNA replication | AJ223087 | 2.37 | 2.53 |
| **POLE2- DNA polymerase epsilon small subunit** | DNA replication | AF036898 | 2.50 | 2.40 |
| **POLD2-DNA polymerase delta 2, regulatory subunit** | DNA replication | Z72486 | 2.42 | 1.23 |
| **Ribonucleotide reductase M2** subunit | Provides precursors for DNA synthesis | M14223 | 2.23 | 2.09 |
| **MCMD4** | Initiation of DNA replication | D26089 | 2.23 | 2.23 |
| **P1 (S. cerevisiae)** | Mcm3 homologue. Initiation of DNA replication | X62154 | 1.65 | 2.14 |
| **DHOH-dihydroorotate dehydrogenase** | Mitochondrial inner membrane, fourth step in pyrimidine biosynthesis | AF029667 | 1.70 | 2.06 |
| **NP95** | Involved in DNA replication-linked nuclear events | D87908 | 1.87 | 2.02 |
| **Dihydrofolate reductase** | Required for purine synthesis | J00388 | 2.00 | 1.65 |
| **HMG4** | Non-histone structural protein of chromatine | AF022465 | 1.25 | 2.00 |
| **HMGIC** | Non-histone structural protein of chromatine | X99915 | 0.67 | 0.44 |
| **MtPoIB mitochondrial DNA polymerase accessory subunit** | Nuclear gene encoding mitochondrial protein, replication of mitochondrial genome | AF006072 | 0.32 | 0.74 |
| **H2B and H2A histone** | Structural protein of chromatine | X05862 | 0.30 | 0.67 |
| **H1 histone** | Structural protein of chromatine | J03482 | 1.00 | 0.16 |
| | | | | |

| **Others** | | | | |
|---|---|---|---|---|
| **Name** | **Description** | **Accession number** | **Relative expression level** | |
| | | | **Day 1** | **Day 3** |
| **COX2. Cyclooxigenase2** | Prostaglandin-endoperoxide synthase 2. Mitogen- and cytokine- inducible form | M88242 | 4.55 | 1.25 |
| | | | | |

| ***Plasminogen*** | | | | |
|---|---|---|---|---|
| **tPA** | Plasminogen activator, tissue | J03520 | 0.73 | 7.02 |
| **PAUR1** | Plasminogen activator, urokinase receptor | X62700 | 2.06 | 2.50 |
| **Nexin-I** | Inhibitor of urokinase-type plasminogen activator (uPA) | X70296 | 0.51 | 0.39 |
| | | | | |

| ***Cholesterol*** | | | | |
|---|---|---|---|---|
| **HMG-CoA reductase** | Control of cholesterol biosynthesis, rate-limiting enzyme in steroid synthesis | M62766 | 2.14 | 11.65 |
| **SC5D. sterol-C5-desaturase** | Cholesterol biosynthesis | | 1.07 | 3.00 |
| **DHCR7. 7-dehydrocholesterol reductase** | Cholesterol biosynthesis | AF057368 | 1.93 | 2.85 |
| **LAL. Lysosomal acid lipase** | Intracellular control of cholesterol and trglyceride catabolism | Z31689 | 2.09 | 0.50 |
| | | | | |

| ***Immunophilins*** | | | | |
|---|---|---|---|---|
| **FKBP5. FK506 binding protein 5** | Immunophilin, intracellular receptor for immunosuppressive drug FK506. Peptidyl-prolyl cis/trans isomerase | U16959 | 1.04 | 7.04 |
| **FKBP1b. FK506-binding protein 1b** | Immunophilin, intracellular receptor for immunosuppressive drug FK506. Peptidyl-prolyl cis/trans isomerase | AF060872 | 0.48 | 0.42 |

| | | | | |
|---|---|---|---|---|
| Total RNA was extracted from non-stimulated confluent cells (day 0) and from cells treated with osteogenic stimulus (GP/AA/BMP-2) (+) or GP alone (-) for 1 and 3 days. RNA samples were analyzed by GeneChip microarray and by NPGN and Expressionist software. The numbers represent fold regulation by osteogenic stimulus, relative to time-matched controls. The genes were considered regulated if their expression changed >2-fold, as compared to the time-matched controls. Red - up-regulated genes; black-down-regulated genes. Within each functional group, the genes were sorted according to the level of regulation, from the strongest stimulation to the strongest inhibition, including both days 1 and 3. In rare cases where the gene was both up-and down-regulated at different times, higher regulation level was taken as a sorting criterium. | | | | |

**(b) Table 2. Expression profiles of genes related to osteoblast differentiation and support of osteoclastogenesis.**

| **BMP-2 signaling pathway** | | | | |
|---|---|---|---|---|
| **Name** | **Description** | **Accession number** | **Relative expression level** | |
| | | | **Day 1** | **Day 3** |
| **Gremlin** | Secreted. BMP antagonist | AF045801 | 0.12 | 0.15 |
| **SMAD1** | BMP signaling pathway | U58992 | 1.38 | 2.08 |
| **SMAD6** | TGF-beta/BMP signaling pathway, inhibitory molecule | AF010133 | 2.63 | 1.36 |
| **SMAD7** | TGF-beta/BMP signaling pathway, inhibitory molecule | AF015260 | 2.25 | 0.88 |
| | | | | |

| **BMP-2 target genes** | | | | |
|---|---|---|---|---|
| **DIx2** | Homeobox. Craniofacial patterning and morphogenesis | M80540 | 3.16 | 1.39 |
| **Id1. Inhibitor of DNA binding 1** | HLH transcriptional inhibitor. BMP-2 inducible | M31885 | 4.00 | 0.67 |
| **Id2. Inhibitor of DNA binding 2** | HLH transcriptional inhibitor. BMP-2 inducible | AF077861 | 3.97 | 2.01 |
| **Id3. Inhibitor of DNA binding 3** | HLH transcriptional inhibitor. BMP-2 inducible | M60523 | 3.01 | 3.25 |
| **Id4** | HLH transcriptional inhibitor | AJ001972 | 0.49 | 0.13 |
| **JunB** | BMP target gene | U20735 | 5.00 | 6.05 |
| | | | | |

| **Related to osteoblast differentiation** | | | | |
|---|---|---|---|---|
| **Alkaline phosphatase** | OB differentiation marker | J02980 | 8.10 | 43.75 |
| **BMP-4** | TGF-beta superfamily. Stimulates OB differentiation | L47480 | 0.44 | 0.77 |
| **Bone sialoprotein** | Integrin binding sialoprotein | L20232 | 1.00 | 12.85 |
| **Fosl1 (Fra-1) Fos-like antigen 1** | AP-1 component. Involved in OB and OC differentiation | AF017128 | 3.40 | 1.00 |
| **LEPR. Leptin receptor** | Cytokine family of receptors | U42467 | 1.00 | 4.95 |
| **LRP5. Low density lipoprotein receptor-related protein 5** | Component of the Wnt receptor complex. | AF064984 | 0.25 | 0.32 |
| **Msx1** | Homeobox domain, msh-like 1. Early phase of OB differentiation | X14759 | 1.05 | 0.25 |
| **Msx2** | Homeobox domain, msh-like 2. Early phase of OB differentiation | X59252 | 4.20 | 1.30 |
| **Osteocalcin** | Bone gamma carboxyglutamate protein 1 | L24431 | 1.00 | 10.20 |
| **PTHR-Parathyroid hormone receptor** | GPCR | X78936 | 2.55 | 33.85 |
| **ROR alpha. RAR-related orphan receptor alpha** | Orphan nuclear receptor | U53228 | 1.55 | 2.60 |
| | | | | |

| **Stimulation of osteoclastogenesis** | | | | |
|---|---|---|---|---|
| **M-CSF** | Macrophage colony-stimulating factor. Stimulates osteoclastogenesis. | M21952 | 0.74 | 0.41 |
| **EGR1/Krox 24. Early growth response 1** | Up-regulated in OB after mechanical loading; Egr1-/-mice: induced production of MCSF, stimulation of osteoclast. | M28845 | 0.33 | 3.62 |
| **OPG. Osteoprotegerin** | Tumor necrosis factor receptor superfamily, member 11b. Inhibits OC genesis | U94331 | 1.27 | 3.84 |
| **Parathyroid hormone-like peptide (PTHRP)** | Supports osteoclastogenesis, osteolytic bone metastases and cartilage-matrix degradation during endochondral bone formation | M60057 | 0.23 | 0.18 |
| **Small inducible cytokine A7** | Chemotactic factor for monocytes | X70058 | 0.74 | 0.45 |

| | | | | |
|---|---|---|---|---|
| Selected data from Table 1, grouped according to their known role in regulating bone homeostasis, osteoblasts and osteoclasts. Red - up-regulated genes; black - down-regulated genes. | | | | |

**(c) Table 3. Expression profiles of components of TGFβ signaling pathway.**

| ***Ligands*** | | | | | | | |
|---|---|---|---|---|---|---|---|
| **Name** | **Description** | **Accession number** | **Day 0** | **Day 1-** | **Day 1+** | **Day 3-** | **Day 3+** |
| TGF-beta1 | TGF-beta family | AJ009862 | 37 | 34 | 155 | 82 | 292 |
| TGF-beta2. | TGF-beta family | X57413 | 74 | 78 | 74 | 97 | 42 |
| TGF-beta3 | TGF-beta family | M32745 | 685 | 700 | 1467 | 502 | 562 |

| ***Receptors*** | | | | | | | |
|---|---|---|---|---|---|---|---|
| **Name** | **Description** | **Accession number** | **Day 0** | **Day 1-** | **Day 1+** | **Day 3-** | **Day 3+** |
| TGF-beta receptor | I TGF-beta receptor | L15436 | 74.5 | 43.5 | 59.5 | 28.4 | 55.3 |
| TGF-beta receptor | II TGF-beta receptor | D32072 | A | A | A | A | A |

| ***Effectors*** | | | | | | | |
|---|---|---|---|---|---|---|---|
| **Name** | **Description** | **Accession number** | **Day 0** | **Day 1-** | **Day 1+** | **Day 3-** | **Day 3+** |
| SMAD2 | TGF-beta activated SMAD | Not on the chip | | | | | |
| SMAD 3 | TGF-beta activated SMAD | AB008192 | A | A | A | A | A |
| SMAD4 | Common SMAD | Not on the chip | | | | | |
| SMAD6 | Inhibitory SMAD | AF010133 | 270 | 276 | 727 | 204 | 277 |
| SMAD7 | Inhibitory SMAD | AF015260 | 114 | 107 | 241 | 104 | 92 |

| ***Regulated target genes*** | | | | | | | |
|---|---|---|---|---|---|---|---|
| **Name** | **Description** | **Accession number** | **Day 0** | **Day 1-** | **Day 1+** | **Day 3-** | **Day 3+** |
| TIEG | TGF beta inducible. Transcriptional repressor | AF064088 | 41 | 66 | 144 | 46 | 88 |
| Tenascin C | Extracellular matrix glycoprotein. Secreted by OB. Stimulate OB differentiation. TGF-beta induced | X56304 | 666 | 883 | 2238 | 721 | 2038 |
| TGFBI. Kerato-epithelin | Extracellular adhesion molecule, binds to Col 1,2 and 4, TGF beta induced | L19932 | 114 | 131 | 457 | 79 | 162 |
| SSTR2. | GPCR. TGF beta | AF008914 | 20 | 20 | 49 | 20 | 57 |
| somatostatin receptor type 2 | induced | | | | | | |

| | | | | | | | |
|---|---|---|---|---|---|---|---|
| Selected data from Table 1, grouped according to their known role in TGF-β signaling. The numbers represent absolute expression levels on microarrays. Day 1, 3+ - samples treated with osteogenic stimulus, Day 1, 3- - time-matched controls. A - absent (signal <20). Red - up-regulated genes; black - down-regulated genes. | | | | | | | |

**(d) Table 4. Expression profiles of components of Wnt signaling pathway.**

| ***Ligands*** | | | | | | |
|---|---|---|---|---|---|---|
| **Name** | **Accession number** | **Day 0** | **Day 1-** | **Day 1+** | **Day 3-** | **Day 3+** |
| Wnt1 | M11943 | A | A | 46 | A | A |
| Wnt2 | Not on the chip | | | | | |
| Wnt2B | AF070988 | A | A | A | A | A |
| Wnt3 | M32502 | A | A | A | A | A |
| Wnt3A | X56842 | A | A | A | A | A |
| Wnt 4 | M89797 | A | A | A | A | A |
| Wnt 5A | M89798 | A | A | A | A | A |
| Wnt 5B | M89799 | 40 | A | 34 | 26 | 39 |
| Wnt6 | M89800 | 24 | 23 | 40 | 27 | 104 |
| Wnt 7A | M89801 | A | A | A | A | A |
| Wnt7B | Al852662 | A | A | A | A | A |
| Wnt8B | Not on the chip | | | | | |
| Wnt8D | Z68889 | A | A | A | A | A |
| Wnt10A | U61969 | 28 | A | 32 | A | 136 |
| Wnt 10B | U61970 | 79 | 71 | 76 | 42 | 65 |
| Wnt 11 | X70800 | A | A | A | A | A |

| ***Receptors*** | | | | | | |
|---|---|---|---|---|---|---|
| **Name** | **Accession number** | **Day 0** | **Day 1-** | **Day 1+** | **Day 3-** | **Day 3+** |
| Frizzled homologue 1 | AF054623 | 77.9 | 62.6 | 65.8 | A | A |
| Frizzled homologue 2 | AW123618 | 894.5 | 809.4 | 655.1 | 786.7 | 630.4 |
| Frizzled homologue 3 | U43205 | A | A | A | A | A |
| Frizzled homologue 4 | U43317 | A | A | A | A | A |
| Frizzled homologue 5 | Not on the chip | | | | | |
| Frizzled homologue 6 | U43319 | A | A | A | A | A |
| Frizzled homologue 7 | U43320 | A | A | A | A | A |
| Frizzled homologue 8 | U43321 | 150.7 | 102.1 | 96.7 | 130.5 | 79.2 |
| Frizzled homologue 9 | Y17709 | A | A | 26 | 45 | 30 |

| **Secreted frizzled-related sequence proteins (sFRPs)** | | | | | | |
|---|---|---|---|---|---|---|
| sFRP1 | U88566 | A | A | A | A | A |
| sFRP2 | U88567 | 3698 | 3772.998 | 3539.999 | 3689.001 | 1266 |
| sFRP4 | AF117709 | A | A | A | A | A |

| ***Low density lipoprotein receptors*** | | | | | | |
|---|---|---|---|---|---|---|
| **Name** | **Accession number** | **Day 0** | **Day 1-** | **Day 1+** | **Day 3-** | **Day 3+** |
| LDLR | Z19521 | 266.4 | 352.8 | 587.9 | 381.1 | 548.5 |
| LRP1 | X67469 | 871.5 | 783.5 | 653.2 | 1167.2 | 447.6 |
| LRP2 | AW259788 | A | A | A | A | A |
| LRP4 | AB013874 | A | A | A | A | A |
| LRP5 | AF064984 | 48 | 80 | A | 62 | M |
| LRP6 | AF074265 | 54.8 | 22.8 | A | 35.8 | 21.3 |

| ***Receptor antagonists*** | | | | | | |
|---|---|---|---|---|---|---|
| **Name** | **Accession number** | **Day 0** | **Day 1-** | **Day 1+** | **Day 3-** | **Day 3+** |
| Dickkopf 1 | AF030433 | A | A | A | A | A |
| Dickkopf 2 | AJ243963 | A | A | A | A | A |
| Dickkopf 3 | AJ243964 | 1078.7 | 605.5 | 517.8 | 609.7 | 327.9 |

| ***Effects*** | | | | | | |
|---|---|---|---|---|---|---|
| **Name** | **Accession number** | **Day 0** | **Day 1-** | **Day 1+** | **Day 3-** | **Day 3+** |
| Beta catenin | M90364 | 2311.6 | 2312.3 | 1530.1 | 2044.6 | 2201.1 |
| Axin | AF009011 | 264.4 | 349.2 | 353.1 | 406.8 | 390.6 |
| APC | M88127 | 40.2 | 48.1 | 50.9 | 32.7 | 31.5 |
| Glycogen synthase kinase 3 | Not on the chip | | | | | |
| Lymphoid enhancer binding factor 1 (LEF1) | D16503 | A | A | A | A | 97 |
| TCF4. Transcription factor 4 | AF107298 | 156 | 136 | 142 | 60 | 122 |

| ***Regulated target genes*** | | | | | | |
|---|---|---|---|---|---|---|
| **Name** | **Accession number** | **Day 0** | **Day 1-** | **Day 1+** | **Day 3-** | **Day 3+** |
| T-cell transcription factor 7 (TCF7) | X61385 | 28 | 29 | 27 | A | 165 |

| | | | | | | |
|---|---|---|---|---|---|---|
| Selected data from Table 1, grouped according to their known role in Wnt signaling. The numbers represent absolute expression levels on microarrays. Day 1, 3+ - samples treated with osteogenic stimulus, Day 1, 3- - time-matched controls. A - absent (signal <20). M - marginal signal. | | | | | | |

**(e) Table 5. Expression profiles of components of Notch signaling pathway.**

| ***Ligands*** | | | | | | |
|---|---|---|---|---|---|---|
| **Name** | **Accession number** | **Day 0** | **Day 1-** | **Day 1+** | **Day 3-** | **Day 3+** |
| Delta-like homologue 1 | X80903 | A | A | A | A | A |
| Delta-like homologue 2 | Not on the chip | | | | | |
| Delta-like homologue 3 | AF068865 | A | A | A | A | A |
| Jagged 1 | Not on the chip | | | | | |
| Jagged 2 | Y14331 | A | A | A | A | A |

| ***Receptors*** | | | | | | |
|---|---|---|---|---|---|---|
| **Name** | **Accession number** | **Day 0** | **Day 1-** | **Day 1+** | **Day 3-** | **Day 3+** |
| Notch homologue 1 | Z11886 | 64 | 91 | 49 | 157 | 67 |
| Notch homologue 2 | D32210 | A | A | A | A | A |
| Notch homologue 3 | X74760 | 227.2 | 135.2 | 50.1 | 249.2 | 66.6 |
| Notch homologue 4 | Not on the chip | | | | | |

| ***Notch regulation*** | | | | | | |
|---|---|---|---|---|---|---|
| **Name** | **Accession number** | **day 0** | **day 1-** | **day 1+** | **day 3-** | **day 3+** |
| ADAM10 | AF011379 | 123 | 75 | 65 | 31 | 72 |
| Furring | Not on the chip | | | | | |
| Presenilin 1 | L42177 | 350 | 340 | 352 | 344 | 346 |
| Presenilin 2 | U57325 | 40 | 28 | 21 | 61 | 34 |
| Lunatic fringe homologue | AF015768 | A | A | 64.1 | A | 36.2 |
| Radical fringe homologue | AF015770 | 328.2 | 321.5 | 374.8 | 292.6 | 384.7 |
| Manic fringe homologue | Not on the chip | | | | | |

| ***Effects*** | | | | | | |
|---|---|---|---|---|---|---|
| **Name** | **Accession number** | **Day 0** | **Day 1-** | **Day 1+** | **Day 3-** | **Day 3+** |
| RBPJk | X17459 | 374 | 213 | 94 | 327 | 113 |

| ***Direct target genes*** | | | | | | |
|---|---|---|---|---|---|---|
| **Name** | **Accession number** | **Day 0** | **Day 1-** | **Day 1+** | **Day 3-** | **Day 3+** |
| HES-1 | D16464 | 135 | 123 | 126 | 72 | 134 |
| HES-2 | AB009967 | A | A | A | A | A |
| HES-3 | D32200 | A | A | A | A | A |
| HES-4 | Not on the chip | | | | | |
| HES-5 | D32132 | A | A | A | A | A |
| HES-6 | AW048812 | 65 | 48 | 43 | 63 | 56 |
| Hey-1 | AJ243895 | A | A | 161 | A | 66 |
| Hey-2 | Not on the chip | | | | | |
| Hey-L | Not on the chip | | | | | |

| | | | | | | |
|---|---|---|---|---|---|---|
| Selected data from Table 1, grouped according to their known role in TGF-β signaling. The numbers represent absolute expression levels on microarrays. Day 1, 3+ - samples treated with osteogenic stimulus, Day 1, 3- - time-matched controls. A - absent (signal <20). | | | | | | |

### Numbered Embodiments of the Invention

1. A method of screening for an agent that modulates the differentiation into osteoblasts, comprising: (a) preparing a first gene or gene family expression profile of a cell population comprising MC3T3-E1 or MC3T3-1 b cells and/or assaying an activity of a protein encoded by at least one gene or a member of a gene family of Table 1 of a cell population comprising MC3T3-E1 or MC3T3-1 b cells; (b) exposing the cell population to the agent; (c) preparing second gene or gene family expression profile of the agent exposed cell population and/or assaying an activity of a protein encoded by at least one gene or a member of a gene family of Table 1 of the exposed cell population; and (d) comparing the first and second expression profiles or first and second activities to an expression profile and/or an activity of an osteblastic differentiated MC3T3-E1 or MC3T3-1b cell population.
2. The method of embodiment 1, wherein the gene expression profiles comprise the expression levels for a set of genes that are differentially regulated in MC3T3-E1 or MC3T3-1 b cells compared to an osteblastic differentiated MC3T3-E1 or MC3T3-1 b cell population.
3. The method of embodiment 1, wherein the agent modulates the level of expression or activity for at least one gene in the MC3T3-E1 or MC3T3-1 b cell population to the expression level found in an osteblastic differentiated MC3T3-E1 or MC3T3-1 b cell population.
4. The method of embodiment 1, wherein the gene expression profiles or activity level comprise the expression or activity levels in a cell of at least two genes or members of a gene family in Table 1.
5. The method of embodiment 1, wherein the gene is Hey1.
6. A method of diagnosing a condition characterized by abnormal deposition of bone tissue, comprising detecting in a tissue sample the level of expression of and/or activity of a protein encoded by at least one gene or member of a gene family of Table 1, wherein differential expression or activity of the gene or member of a gene family is indicative of abnormal bone tissue deposition.
7. A method of monitoring the treatment of a patient with a condition characterized by abnormal bone tissue deposition, comprising: (a) administering a pharmaceutical composition to the patient; (b) preparing a gene expression profile from a cell or tissue sample from the patient and/or assaying an activity of a protein encoded by at least one gene or a member of a gene family of Table 1; and (c) comparing the patient expression profile or activity to an expression profile or activity from a MC3T3-E1 or MC3T3-1b cell population or an osteblastic differentiated MC3T3-E1 or MC3T3-1 b cell population.
8. A method of diagnosing a condition characterized by an abnormal rate of formation of osteoblasts, comprising detecting in a tissue sample a level of expression of and/or activity of a protein encoded by at least one gene or member of a gene family from Table 1, wherein differential expression and/or activity of the gene or member of a gene family is indicative of an abnormal rate of formation of osteoblasts.
9. A method of monitoring the treatment of a patient with a condition characterized by abnormal rate of formation of osteoblasts, comprising: (a) administering a pharmaceutical composition to the patient; (b) preparing a gene expression profile and/or assaying an activity of at least one gene or member of a gene family from Table 1 in a cell or tissue sample from the patient; and (c) comparing the patient gene expression profile and/or activity to a gene expression profile or activity from a MC3T3-E1 or MC3T3-1b cell population or an osteblastic differentiated MC3T3-E1 or MC3T3-1 b cell population.
10. A method of diagnosing osteoporosis in a patient, comprising detecting the level of expression and/or activity in a tissue sample of at least one gene or member of a gene family from Table 1; wherein differential expression or activity is indicative of osteoporosis.
11. A method of monitoring the treatment of a patient with osteoporosis, comprising: (a) administering a pharmaceutical composition to the patient; (b) preparing a gene expression profile and/or assaying an activity of at least one gene or member of a gene family of Table 1 in a cell or tissue sample from the patient; and (c) comparing the patient gene expression profile and/or activity to a gene expression profile or activity in a MC3T3-E1 or MC3T3-1 b cell population and/or an osteblastic differentiated MC3T3-E1 or MC3T3-1 b cell population.
12. A method of screening for an agent capable of ameliorating the effects of osteoporosis, comprising: (a) exposing a cell to the agent; and (b) detecting the expression and/or activity level of one or more genes or members of a gene family of Table 1.
13. A method of monitoring the progression of bone tissue deposition in a patient, comprising detecting the level of expression and/or activity in a tissue sample of at least one gene or member of a gene family from Table 1; wherein differential expression and/or activity is indicative of bone tissue deposition.
14. A method of screening for an agent capable of modulating the deposition of bone tissue, comprising: (a) exposing a cell to the agent; and (b) detecting the expression and/or activity level of at least one gene or member of a gene family of Table 1.
15. The method of any one of embodiments 1-14, wherein expression and/or activity levels of at least 2 genes are detected.
16. The method of any one of embodiments 1-14, wherein expression and/or activity levels of at least 3 genes are detected.
17. The method of any one of embodiments 1-15, wherein expression and/or activity levels of at least 4, 5, 6, 7, 8, 9, 10 or 11 genes are detected.
18. The method of any one of embodiments 1-14, wherein expression and/or activity levels of all the genes in Table 1 are detected.
19. The method of any one of embodiments 1-14, wherein expression and/or activity levels of all the genes in Table 2 are detected.
20. The method of any one of embodiments 1-14, wherein expression and/or activity levels of all the genes in Table 3 are detected.
21. A composition comprising at least two oligonucleotides, wherein each of the oligonucleotides comprises a sequence that specifically hybridizes to a gene or member of a gene family of Table 1.
22. The composition according to embodiment 21, wherein the composition comprises at least 3 oligonucleotides, wherein each of the oligonucleotides comprises a sequence that specifically hybridizes to a gene or member of a gene family of Table 1.
23. The composition according to embodiment 21, wherein the composition comprises at least 5 oligonucleotides, wherein each of the oligonucleotides comprises a sequence that specifically hybridizes to a gene or member of a gene family of Table 1.
24. The composition according to embodiment 21, wherein the composition comprises at least 7 oligonucleotides, wherein each of the oligonucleotides comprises a sequence that specifically hybridizes to a gene or member of a gene family of Table 1.
25. The composition according to embodiment 21, wherein the composition comprises at least 10 oligonucleotides, wherein each of the oligonucleotides comprises a sequence that specifically hybridizes to a gene or member of a gene family of Table 1.
26. The composition according to any one of embodiments 21-25, wherein the oligonucleotides are attached to a solid support.
27. The composition according to embodiment 25, wherein the solid support is selected from a group consisting of a membrane, a glass support, a filter, a tissue culture dish, a polymeric material and a silicon support.
28. A solid support to which is attached at least two oligonucleotides, wherein each of the oligonucleotides comprises a sequence that specifically hybridizes to a gene or member of a gene family of Table 1.
29. The solid support according to embodiment 28, wherein at least one oligonucleotide is attached covalently.
30. The solid support according to embodiment 28, wherein at least one oligonucleotide is attached non-covalently.
31. The solid support of embodiment 28, wherein the solid support is an array comprising at least 10 different oligonucleotides in discrete locations per square centimeter.
32. The solid support of embodiment 28, wherein the array comprises at least 100 different oligonucleotides in discrete locations per square centimeter.
33. The solid support of embodiment 28, wherein the array comprises at least 1000 different oligonucleotides in discrete locations per square centimeter.
34. The solid support of embodiment 28, wherein the array comprises at least 10000 different oligonucleotides in discrete locations per square centimeter.
35. A computer system comprising: (a) a database containing information identifying the expression and /or activity level in osteoblasts of a set of genes comprising one or more genes or members of a gene family in Table 1; and (b) a user interface to view the information.
36. The computer system of embodiment 35, wherein the database further comprises sequence information for the genes or gene families.
37. The computer system of embodiment 35, wherein the database further comprises information identifying the expression and/or activity level in MC3T3-E1 and/or MC3T3-1 b cells of at least one gene or member of a gene family of Table 1.
38. The computer system of embodiment 35, wherein the database further comprises information identifying the expression level a set of genes indicative of a condition characterized by abnormal bone tissue deposition.
39. The computer system of any of embodiments 35-38, further comprising records including descriptive information from an external database, which information correlates said genes to records in the external database.
40. The computer system of embodiment 39, wherein the external database is GenBank.
41. The method of using a computer system of any one of embodiments 35-40 to present information identifying the expression level in a tissue or cell of a set of genes comprising at least two of the genes or members of gene families in Table 1, comprising: (a) comparing the expression level of at least one gene or member of a gene family in Table 1 in the tissue or cell to the level of expression of the gene in the database.
42. The method of embodiment 41, wherein the expression levels of at least two genes are compared.
43. The method of embodiment 41, wherein the expression levels of at least five genes are compared.
44. The method of embodiment 41, wherein the expression levels of at least ten genes are compared.
45. The method of embodiment 41, further comprising the step of displaying the level of expression of at lest one gene in the tissue or cell sample compared to the expression level in osteblastic differentiated MC3T3-E1 cells and/or MC3T3-1 b cells.

## Claims

1. A method of screening for an agent that modulates the differentiation into osteoblasts, comprising (a) detecting sFRP2 gene expression of a cell population comprising MC3T3-E1 or MC3T3-1 b cells and/or assaying an activity of a protein encoded by at least sFRP2 gene of a cell population comprising MC3T3-E1 or MC3T3-1 b cells; (b) exposing the cell population to the agent; (c) detecting sFRP2 gene expression of the agent exposed cell population and/or assaying an activity of a protein encoded by at least sFRP2 gene of the exposed cell population and (d) comparing the sFRP2 expression or sFRP2 activities obtained in (a) and (c) to sFRP2 expression and/or sFRP2 activity of an osteoblastic differentiated MC3T3-E1 or MC3T3-1 b cell population.

2. The method of claim 1, wherein the agent modulates the level of expression or activity for sFRP2 gene in the MC3T3-E1 or MC3T3-1 b cell population to the expression level found in an osteoblastic differentiated MC3T3- E1 or MC3T3-1b cell population.

3. A method of diagnosing a condition **characterized by** abnormal deposition of bone tissue, comprising detecting in a tissue sample the level of expression of and/or activity of a protein encoded by sFRP2 gene, wherein altered expression of or activity of a protein encoded by the sFRP2 gene is indicative of abnormal bone tissue deposition.

4. A method of diagnosing a condition **characterized by** an abnormal rate of formation of osteoblasts, comprising detecting in a tissue sample a level of expression of and/or activity of a protein encoded by at least sFRP2 gene, wherein altered expression and/or activity of a protein encoded by the sFRP2 gene is indicative of an abnormal rate of formation of osteoblasts.

5. A method of diagnosing osteoporosis in a patient, comprising detecting the level of expression and/or activity in a tissue sample of at least sFRP2 gene; wherein altered expression or activity is indicative of osteoporosis.

6. A method of screening for an agent capable of ameliorating the effects of osteoporosis, comprising: (a) exposing a cell to the agent; and (b) detecting the expression and/or activity level of sFRP2 gene or the protein encoded by sFRP2 gene.

7. A method of monitoring the progression of bone tissue deposition in a patient, comprising detecting the level of expression in a tissue sample of at least sFRP2 gene ; wherein altered expression is indicative of bone tissue deposition.

8. An in vitro method of screening for an agent capable of modulating the deposition of bone tissue, comprising: (a) exposing a cell to the agent; and (b) detecting the expression and/or activity level of at least sFRP2 gene or protein encoded by sFRP2 gene.

9. A sFRP2 modulating agent for use as a medicament.

10. A sFRP2 modulating agent for use in the treatment of conditions **characterized by** abnormal rate of formation of osteoblasts, abnormal bone tissue deposition or bone loss diseases such as osteoporosis.

11. The sFRP2 modulating agent of claim 9 or 10, wherein said sFRP2 modulating agent alters the expression of sFRP2 gene and/or alters the activity of a protein encoded by sFRP2 gene.

12. The sFRP2 modulating agent of any of claims 9-11, wherein said sFRP2 modulating agent is an antibody specific for the protein encoded by sFRP2 gene.

13. Use of a sFRP2 modulating agent in the preparation of a medicament for the treatment of conditions **characterized by** abnormal rate of formation of osteoblasts, abnormal bone tissue deposition or bone lose diseases such as osteoporosis.

14. An antibody specific for the protein encoded by sFRP2 gene as a probe to monitor differential expression of sFRP2 gene in different cell populations.

15. Use of an antibody specific for the protein encoded by sFRP2 gene as a probe to monitor differential expression of sFRP2 gene in different cell populations.

16. An inhibitor of Wnt signalling pathway for use in the treatment of conditions
**characterized by** abnormal rate of formation of osteoblasts, abnormal mineralization process or bone loss diseases such as osteoporosis.

17. The inhibitor of Wnt signalling pathway according to claim 16, capable of reducing sFRP2 transcriptional activity.
